# EUROPEAN PATENT APPLICATION

(11) **EP 2 450 702 A2**
(43) Date of publication of application: **09.05.2012**
(21) Application number: 11188307.0
(22) Date of filing: 28.01.2008
(51) Int. Cl.: G01N 31/00, G01N 33/543, G01N 33/574, G01N 33/68

(54) **Methods of detecting autoantibodies for diagnosing and characterizing disorders**

(30) Priority: 26.01.2007 US 897641 P
(62) Divisional of application: 08728418.8
(71) Applicant: University Of Louisville Research Foundation, Inc., Louisville, KY 40202 (US)
(72) Inventor: Taylor, Douglas D., Louisville, Kentucky 40245 (US); Gercel-Taylor, Cicek, Louisville, Kentucky 40245 (US)
(74) Representative: Wallis, Naomi Rachel

(57) **Abstract**

Methods for detecting and/or quantitating levels of autoantibodies in subjects are provided. Methods for diagnosing and/or characterizing a disorder associated with autoantibody production are further provided. In some embodiments, the disorder diagnosed and/or characterized can be a cancer or an infertility disorder.

## Description

### RELATED APPLICATIONS

The presently disclosed subject matter claims the benefit of U.S. Provisional Patent Application Serial No. 60/897,641, filed January 26, 2007; the disclosure of which is incorporated herein by reference in its entirety.

### GOVERNMENT INTEREST

This presently disclosed subject matter was made, in part, with U.S. Government support under Grant No. CA104651 awarded by the National Cancer Institute. Thus, the U.S. Government has certain rights in the presently disclosed subject matter.

### TECHNICAL FIELD

The presently disclosed subject matter relates to diagnosing and characterizing disorders in a subject by detection of autoantibodies. In particular, the presently disclosed subject matter relates to utilizing antigens, including in some embodiments antigens isolated from cell-produced exosomes, to detect autoantibodies in subjects for diagnosing or characterizing cancer and/or infertility and/or other disorders in the subject.

### BACKGROUND

Many disorders can be more effectively treated and/or prevented if they are diagnosed at an early stage. This is particularly true for cancer. For example, Stage I ovarian cancer can be cured in 90% of cases, while five-year survival for patients with advanced disease (Stages III and IV) is less than 21 %. Thus, prospects for significant improvement in cancer survival reside in early diagnosis. Similarly, other disorders could potentially be diagnosed earlier and/or prognosis for treatments determined better if more sensitive early diagnostic tests were available. As another particular example, infertility disorders are notoriously difficult to diagnose and define in advance of reported difficulties with initiating or maintaining to term a viable pregnancy. More sensitive and specific diagnostic assays could provide better diagnosis of the infertility disorders and prognosis for infertility treatments.

Current diagnostic assays for many disorders, including cancer for example, are antigen-based and rely on the detection of circulating proteins associated with the disorder. These assays rely on the expression, synthesis, and release of specific proteins by cells (e.g., tumor cells) either by active secretion or shedding or as a consequence of cell death (either necrosis or apoptosis). These antigenic proteins must "escape" the primary site of disease, saturate the antigen-processing capacity of the individual's immune components, gain access to the circulation, and reach a sufficient steady-state concentration to be detected by enzyme- or radiolabel based immunoassays. These events usually occur well after the initial establishment of disease (e.g., a neoplastic transformation event and tumor foci development).

Thus, current antigen-based assays cannot truly detect early stages of a disorder of interest. To significantly improve diagnostic assays for disorders of interest, there is an unmet need for a new approach to detection of disorders that is more sensitive to the presence of markers of disease early in the establishment of the disorder.

### SUMMARY

This Summary lists several embodiments of the presently disclosed subject matter, and in many cases lists variations and permutations of these embodiments. This Summary is merely exemplary of the numerous and varied embodiments. Mention of one or more representative features of a given embodiment is likewise exemplary. Such an embodiment can typically exist with or without the feature(s) mentioned; likewise, those features can be applied to other embodiments of the presently disclosed subject matter, whether listed in this Summary or not. To avoid excessive repetition, this Summary does not list or suggest all possible combinations of such features.

In some embodiments of the presently-disclosed subject matter, a method for detecting and/or quantitating a level of autoantibodies in a subject is provided. The method comprises in some embodiments, providing a biological sample comprising or suspected of comprising autoantibodies from a subject and contacting an antigen with the sample. The antigen can comprise an autoantibody immunoreactive peptide isolated from an exosome. The method further comprises detecting and/or quantitating a level of the autoantibodies in the sample immunoreactive to the antigen. In some embodiments, the autoantibodies are associated with a cancer or infertility disorder.

The presently-disclosed subject matter further provides in some embodiments a method for diagnosing a disorder associated with autoantibody production in a subject. The method comprises in some embodiments, providing a biological sample comprising or suspected of comprising autoantibodies from a subject and contacting an antigen with the sample. The antigen can comprise an autoantibody immunoreactive peptide isolated from an exosome. The method further comprises detecting autoantibodies in the sample immunoreactive to the antigen and comparing a level of autoantibody immunoreactivity to the antigen with a reference level to diagnose the disorder in the subject.

The presently-disclosed subject matter still further provides in some embodiments a method for characterizing a disorder associated with autoantibody production in a subject. The method comprises in some embodiments, providing a biological sample comprising autoantibodies from a subject and contacting an antigen with the sample. The antigen can comprise an autoantibody immunoreactive peptide isolated from an exosome. The method further comprises detecting the autoantibodies in the sample immunoreactive to the antigen and quantitating a level of autoantibody immunoreactivity to the antigen to thereby characterize the disorder in the subject.

In some embodiments of the methods for diagnosing or characterizing disorders, the disorder is a cancer or an infertility disorder. In some embodiments of the methods disclosed herein, the disorder is an epithelial cancer or an adenocarcinoma. Further, in some embodiments, the antigen comprises a cancer antigen peptide selected from the group consisting of p53, p63, p73, mdm-2, procathepsin-D, B23, C23, PLAP, cerB/HER2, NY-ESO-1, SCP1, SSX-1, SSX-2, SSX-4, HSP10, HSP27, HSP60, HSP90, GRP78, HoxA7, HoxB7, EpCAM, c-ras, mesothelin, survivin, a mucin, EGF kinase, c-myc, nucleophosmin, and TAG 72. In some embodiments of the method, the disorder is an infertility disorder selected from the group consisting of premature ovarian failure (POF), polycystic ovary syndrome (PCOS), endometriosis, preeclampsia, preterm birth, intrauterine growth restriction, and recurrent pregnancy loss.

In some embodiments of the methods, the biological sample comprises milk, blood, serum, plasma, ascites, cyst fluid, pleural fluid, tears, urine, saliva, tissue, or combinations thereof. Further, in some embodiments, the subject is a mammal.

In some embodiments of the presently-disclosed methods, the exosome is isolated from a cell, which can in some embodiments be a cultured cell. In some embodiment, the cell is a cancer cell, such as for example, an ovarian cancer cell, a cervical cancer cell, a breast cancer cell, an endometrial cancer cell, a colon cancer cell, a prostate cancer cell, a lung cancer cell, a melanoma cell, a pancreatic cancer cell, or a choriocarcinoma cell. In some particular embodiments, the cell is a UL-1 cell, a UL-2, a UL-3 cell, or a UL-6 cell. In some particular embodiments, the cell is a placental cell.

In some embodiments of the presently-disclosed methods, the detecting comprises a technique selected from the group consisting of ELISA, RIA, multiplex immunoassay, immunoprecipitation and Western blotting.

The presently-disclosed subject matter still further provides in some embodiments a kit for detecting autoantibodies in a sample. In some embodiments, the kit comprises an autoantibody immunoreactive peptide antigen and a container for containing the antigen. The antigen can be isolated from an exosome in some embodiments. In some embodiments, the antigen is attached to a support. In some embodiments, the support is a microtiter plate, a membrane (nitrocellulose, PVDF or similar material), a polystyrene bead, a test tube or a dipstick. In some embodiments, the kit comprises an antibody preparation that binds to an autoantibody. Further, in some embodiments, the antibody preparation comprises a detectable label. Still further, in some embodiments the detectable label comprises a radiolabel, an enzyme, biotin, a dye, a fluorescent tag label, a hapten or a luminescent label.

Accordingly, it is an object of the presently disclosed subject matter to provide methods of detecting autoantibodies for diagnosing and characterizing disorders. This object is achieved in whole or in part by the presently disclosed subject matter.

An object of the presently disclosed subject matter having been stated hereinabove, and which is achieved in whole or in part by the presently disclosed subject matter, other objects and advantages will become evident to those of ordinary skill in the art after a study of the following description of the presently disclosed subject matter, Figures, and non-limiting Examples.

### BRIEF DESCRIPTION OF THE DRAWINGS

Figure 1 is a graph showing correlation of tumor reactive immunoglobulins in cancer patients with stage of disease, compared with normal non-tumor-bearing controls.
Figures 2A and 2B are photographs showing immunoreactivity of immunoglobulins present in the sera of representative ovarian cancer patients (2A = stage II and 2B = stage IV) with cellular antigens derived from normal ovarian epithelium (NO) and 3 ovarian tumor cell lines (UL1, UL3, and UL6). Boxes designate areas of interest.
Figures 3A-3D are a series of photographs showing 2-dimensional electrophoretic analyses of recognition pattern differences in patients responding to cisplatin. Figures 3A-3D present a portion of the 2D blots using antigens from UL-6 as targets and sera from patients with Stage IIIc cyst adenocarcinoma of the ovary. Patients A and B (Figures 3A and 3B, respectively) responded to cisplatin, while Patients C and D (Figures 3C and 3D, respectively) failed to respond.
Figures 4A and 4B are graphs showing protein array profiles from 2 ovarian cancer patients. Immunoprecipitated cellular proteins from UL-1 ovarian tumor cell line were separated by RP-HPLC and proteins bound to MAGNAGRAPH membranes. Immunoreactive proteins were identified by incubating the wells with sera from ovarian cancer patients, defined as pixels determined by densitiometry.
Figure 5 is an illustration of a pattern of immunoreactivity by patient-derived tumor-reactive autoantibodies.
Figure 6 is a graph showing percent of sera from normal female volunteers (control), women with benign ovarian disease, and women with invasive ovarian cancer exhibiting autoantibodies reactive with antigens (listed in Table 1) in a protein array.
Figure 7 is a series of photographs showing raw protein microarrays demonstrating reactive IgG in women with early versus late stage ovarian cancer.
Figure 8A is a series of photographs showing reactivity of cervical cancer patient antisera with cellular antigens. Figure 8B is a graph showing reactivity of cervical cancer cell lines derived antigens with patient sera.
Figure 9 is a series of photographs and a graph showing the effect of retinoic acid on the reactivity of soluble antigens released from cervical cancer cells.
Figure 10 is a series of photographs and a graph showing the effect of retinoic acid on the reactivity of cell-associated antigens released from cervical cancer cells.
Figure 11 is a series of graphs showing the immunoreactivity of sera from different patients with various stages of cancer, benign disease, or normal controls against different autoantibody immunoreactive peptide antigens.
Figure 12 is a series of graphs showing the immunoreactivity of sera from control subjects and patients with various different types of cancers against different autoantibody immunoreactive peptide antigens.
Figure 13 is a series of photographs showing differences in antigenic epitopes in recombinant antigens verses natural exosome derived antigens against sera from different cancer patients.
Figure 14 is a graph showing ELISA results of immunoreactivity of patient sera, diagnosed with stage I, II or III endometriosis versus normal controls, against cellular antigens derived from subcellular compartments of the endometrium. Antigens were isolated from the membrane, nuclear, and cytosol fractions of endometrial cells and coupled to wells of microtiter plates.
Figure 15 is series of photographs showing western immunoblots of cellular antigens from endometrium and ovary recognized by autoreactive humoral response.
Figures 16A-16C are a series of photographs showing portions of representative immune recognition of endometrial membrane antigens separated by two-dimensional electrophoresis by sera of patients with stage I I and III endometriosis. Proteins were isolated from Hec-1A, endometrial tumor cell line. Solubilized membrane proteins (100 mg) were loaded to a PH 3-10 isoelectric focusing strip and after running the strip was applied to the top of a 10-20% acrylamide SDS-PAGE gel. After SDS-PAGE, the proteins were transferred to nitrocellulose and immunoblotted.
Figure 17 is a series of photographs showing serologic reactivity patterns of sera obtained from women diagnosed with infertility disorders against cellular antigens derived from the endometrium.
Figure 18 is a series of photographs showing western immunoblots demonstrating the presence of autoantibodies produced during pregnancy by normal term deliveries and recurrent pregnancy loss.

### DETAILED DESCRIPTION

The details of one or more embodiments of the presently disclosed subject matter are set forth in the accompanying description below. Other features, objects, and advantages of the presently disclosed subject matter will be apparent from the detailed description, examples, and claims. All publications, patent applications, patents, and other references cited herein are incorporated by reference in their entirety. In case of conflict, the present specification, including definitions, will control.

Following long-standing patent law convention, the terms "a", "an" and "the" mean "one or more" when used in this application, including in the claims.

Unless otherwise indicated, all numbers expressing quantities of ingredients, reaction conditions, and so forth used in the specification and claims are to be understood as being modified in all instances by the term "about". Accordingly, unless indicated to the contrary, the numerical parameters set forth in this specification and attached claims are approximations that can vary depending upon the desired properties sought to be obtained by the presently disclosed subject matter.

As used herein, the term "about," when referring to a value or to an amount of mass, weight, time, volume, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

Unless defined otherwise, all technical and scientific terms used herein have the same meaning as commonly understood to one of ordinary skill in the art to which the presently disclosed subject matter belongs. Although any methods, devices, and materials similar or equivalent to those described herein can be used in the practice or testing of the presently disclosed subject matter, representative methods, devices, and materials are now described.

It has been determined that certain disorders are characterized by the production of autoantibodies in the afflicted subject. That is, the immune system of the subject is stimulated to produce antibodies against self antigens (as opposed to foreign antigens, such as antigens unique to an invading microorganism). The antigens to which the autoantibodies immunoreact can in some instances have altered epitopes due to changes in primary sequence or post-translational processing (e.g., as can occur in cancer), but can also be immunologically identical to the normal antigen. In either instance, it has been discovered that autoantibody production can be correlated with the presence of a disorder and can even provide information for characterizing the disorder. The presently-disclosed subject matter provides novel methods for detecting and/or quantitating levels of autoantibodies in subjects, which can be correlated with the presence of a disorder in the subject and/or characterization of the disorder. For example, in some embodiments of the presently disclosed subject matter, methods are provides for diagnosing and/or characterizing a cancer or infertility disorder in a subject that is associated with autoantibody production.

### I. Methods For Detecting And Quantitating Autoantibody Levels

The presently disclosed subject matter provides, in some embodiments, methods for detecting and/or quantitating levels of autoantibodies in a subject. These methods can be utilized in some embodiments for diagnosing and/or characterizing disorders in subjects that are associated with autoantibody production.

In some embodiments, the methods comprise providing a biological sample comprising or suspected of comprising autoantibodies from a subject and then contacting an antigen with the sample. The antigen comprises an autoantibody immunoreactive peptide. The method then comprises detecting and/or quantifying a level of the autoantibodies in the sample that are immunoreactive to the antigen. In some embodiments, the biological sample can comprise, for example, milk, blood, serum, plasma, ascites, cyst fluid, pleural fluid, saliva, tears, urine, tissue, or combinations thereof.

Further, In some embodiments of the presently disclosed subject matter, a method for diagnosing a disorder associated with autoantibody production in a subject based on detection of autoantibodies in the subject is provided. In some embodiments, the method comprises providing a biological sample comprising or suspected of comprising autoantibodies from a subject; contacting an antigen with the sample, wherein the antigen comprises an autoantibody immunoreactive peptide; detecting the autoantibodies in the sample immunoreactive to the antigen; and comparing a level of autoantibody immunoreactivity to the antigen with a reference level to diagnose the disorder in the subject.

The terms "diagnosing" and "diagnosis" as used herein refer to methods by which the skilled artisan can estimate and even determine whether or not a subject is suffering from a given disorder or condition. The skilled artisan often makes a diagnosis on the basis of one or more diagnostic indicators, such as for example an autoantibody, the amount (including presence or absence) of which is indicative of the presence, severity, or absence of the condition.

Along with diagnosis, clinical prognosis is also an area of great concern and interest. It is important to know the severity of the disorder (e.g., aggressiveness of cancer cells and the likelihood of tumor recurrence) in order to plan the most effective therapy. Measurement of autoantibodies can be useful in order to separate subjects with good prognosis who will need no further therapy from those more likely might benefit from more intensive treatments.

As such, "making a diagnosis" or "diagnosing", as used herein, is further inclusive of making a prognosis, which can provide for predicting a clinical outcome (with or without medical treatment), selecting an appropriate treatment (or whether treatment would be effective), or monitoring a current treatment and potentially changing the treatment, based on the measure of a diagnostic autoantibody.

Further, in some embodiments of the presently disclosed subject matter, multiple determination of the autoantibodies over time can be made to facilitate diagnosis and/or prognosis. A temporal change in the autoantibody levels can be used to predict a clinical outcome, monitor the progression of the disorder and/or efficacy of appropriate therapies directed against the disorder. In such an embodiment for example, one might expect to see a decrease in the amount of autoantibodies (and potentially one or more additional biomarker(s), if monitored) in a biological sample over time during the course of effective therapy.

Correlating a level, such as an increased level, of autoantibodies with a reference level or "normal level" to diagnose and/or characterize a disorder refers to a comparison of autoantibody levels (quantitative and/or presence or absence) with levels expected (including but not limited to no autoantibody detected) in a subject free of the disorder. A change, such as an increase, over normal levels refers to a result that is changed, e.g. increased, by more than the margin of error inherent in the measurement technique when comparing the sample to a similar disease free sample under otherwise comparable conditions. In some embodiments an increased level of detected autoantibodies in the test subject is by about 10% or greater over a baseline "normal" presence. In some embodiments an increased level of detected autoantibodies in the test subject by about 20% or greater, in some embodiments an increased level of detected autoantibodies in the test subject by about 25% or greater, and in some embodiments an increased level of detected autoantibodies in the test subject by about 50% or greater is an increased presence of autoantibodies, which can be correlated with presence of the disorder in the subject.

Further, in some embodiments of the presently disclosed subject matter, a method for characterizing a disorder associated with autoantibody production in a subject is provided. "Characterizing", as used herein, can refer to detecting the presence of a disorder or determining the severity of a disorder, such as for example determining a cancer stage. In some embodiments, the method comprises providing a biological sample comprising autoantibodies from a subject; contacting an antigen with the sample, wherein the antigen comprises an autoantibody immunoreactive peptide; detecting the autoantibodies in the sample immunoreactive to the antigen; and quantitating a level of autoantibody immunoreactivity to the antigen to thereby characterize the disorder in the subject.

The terms "immunoreact" and "immunoreactive", as used herein and with regard to antibody binding, refer to the specific binding by the variable regions of antibodies to specific epitopes of antigens.

The terms "peptide", "polypeptide", and "protein", which are used interchangeably herein, refer to a polymer of the 20 protein amino acids, or amino acid analogs, regardless of its size or function. Although "protein" is often used in reference to relatively large polypeptides, and "peptide" is often used in reference to small polypeptides, usage of these terms in the art overlaps and varies. The term "peptide" as used herein refers to peptides, polypeptides, and proteins, unless otherwise noted. The terms "protein", "polypeptide" and "peptide" are used interchangeably herein when referring to a gene product. Thus, exemplary polypeptides include gene products, naturally occurring proteins, homologs, orthologs, paralogs, fragments and other equivalents, variants, and analogs of the foregoing.

In some embodiments of the methods disclosed herein, detecting the autoantibodies in the sample can include binding the autoantibodies to an antigen and then detecting either the binding event or the presence of the autoantibody isolated from the biological sample. Exemplary techniques for detecting the autoantibodies include, but are not limited to, enzyme-linked immunosorbent assay (ELISA), radioimmunoassay (RIA), multiplex immunoassay, immunoprecipitation and immunoblotting (including, for example, Western blotting and dot blotting).

Various useful immunodetection methods have been described in the scientific literature, such as Nakamura et al. (In: Handbook of Experimental Immunology (4th Ed.), Weir et al. (eds). Vol.1, Chapter 27, Blackwell Scientific Publ., Oxford, 1987; incorporated herein by reference). Immunoassays, in their most simple and direct sense, are binding assays. Exemplary immunoassays include the various types of ELISAs, RIAs, and multiplex immunoassays. Immunohistochemical detection using tissue sections also is particularly useful. However, it will be readily appreciated that detection is not limited to such techniques, and Western blotting, dot blotting, FACS analyses, precipitin reactions, and the like also can be used in connection with the presently disclosed subject matter.

In general, with regard to the present methods, immunobinding methods include obtaining a sample suspected of containing an antibody and contacting the sample with an antigen (e.g. an autoantibody immunoreactive peptide) in accordance with the present subject matter under conditions effective to allow the formation of immunocomplexes.

Contacting the chosen biological sample with the antigen under conditions effective and for a period of time sufficient to allow the formation of immune complexes (primary immune complexes) is generally a matter of adding the composition to the sample and incubating the mixture for a period of time long enough for the antibodies to form immune complexes with the antigens presented. After this time, the antigen-antibody mixture can be washed to remove any non-specifically bound antibody species, allowing only those antibodies specifically bound within the primary immune complexes to be detected.

In general, the detection of immunocomplex formation can be achieved through the application of numerous approaches. These methods are generally based upon the detection of a label or marker, such as any radioactive, fluorescent, biological or enzymatic tags or labels of standard use in the art. U.S. Patent Nos. concerning the use of such labels include 3,817,837; 3,850,752; 3,939,350; 3,996,345; 4,277,437; 4,275,149; 4,302,534; 4,366,241; 4,637,988; 4,786,594; 5,108,896; 5,229,302; 5,629,164 and 5,691,154 each incorporated herein by reference. Of course, one may find additional advantages through the use of a secondary binding ligand such as a second antibody or a biotin/avidin ligand binding arrangement, as is known in the art.

In some embodiments, the primary immune complexes can be detected by a second binding ligand that has binding affinity for the antigen or the antibody presented in the sample (either specifically or non-specifically (e.g., reactivity to Fc region of the autoantibodies)). In these cases, the second binding ligand can be linked to a detectable label. The second binding ligand is itself often an antibody, which may thus be termed a "secondary" antibody. The primary immune complexes are contacted with the labeled, secondary binding ligand, or antibody, under conditions effective and for a period of time sufficient to allow the formation of secondary immune complexes. The secondary immune complexes are then generally washed to remove any unbound labeled secondary antibodies or ligands, and the remaining label in the secondary immune complexes is then detected.

Other methods include the detection of primary immune complexes by a two step approach. A second binding ligand, such as an antibody, that has binding affinity for the antigen or autoantibody is used to form secondary immune complexes, as described above. The second binding ligand contains an enzyme capable of processing a substrate to a detectable product and, hence, amplifying signal over time. After washing, the secondary immune complexes are contacted with substrate, permitting detection.

Competitive immunodetection can also be used to detect the presence of autoantibodies specific for the test antigens. In this technique, a labeled-antibody is first incubated in solution with the antigen. Signal emitted by the label is measured. This is followed by contacting this antigen/antibody complex with a sample containing or suspected of containing the antibodies of interest. If the sample has antibodies specific to the antigen, they will bind the antigen and competitively displace the labeled-antibody. This can be detected as a drop in intensity of the signal from the label.

In some, but not all, embodiments of the presently disclosed subject matter, the antigens utilized to capture the autoantibodies from the biological sample are isolated from exosomes. The term "isolated", as used herein when applied to a polypeptide, denotes that the polypeptide is essentially free of other cellular or exosomal components with which it is associated in the natural state.

Exosomes are vesicles of endosomal origin that are secreted in the extracellular milieu following fusion of late endosomal multivesicular bodies with the plasma membrane. Cells from various tissue types have been shown to secrete exosomes, such as dendritic cells, B lymphocytes, tumor cells and mast cells, for instance. Exosomes from different origins exhibit discrete sets of proteins and lipid moieties. They notably contain proteins involved in antigen presentation and immunomodulation, suggesting that exosomes play a role in cell-cell communications leading to the modulation of immune responses. Indeed, exosomes from dendritic cells (DC) pulsed with peptides derived from tumor antigens elicit anti-tumor responses in animal model using the matching tumor. However, exosomes derived from cancer cells comprising cancer antigens have been shown to comprise immunosuppressive polypeptides, making unmodified tumor-derived exosomes undesirable and potentially unsafe for use directly in vaccines.

The exosomes of the presently disclosed subject matter are well-suited for producing antigens that can immunoreact with autoantibodies to capture the autoantibodies out of biological samples from subjects because they are produced by cells, rather than artificially-synthesized, and therefore provide antigens that are "natural". That is, the antigens produced by the cells and found in the exosome can be full-length peptides that are processed (e.g., glycosylated) and folded by the cell to a similar extent as antigens experienced by immune cells in a subject. As such, the exosome antigens can be utilized in assays for detecting autoantibodies that can be present in subjects with disorders such as, for example, cancers and infertility disorders. In some embodiments, therefore, the one or more antigens can each comprise a cancer cell antigen and/or infertility disorder antigen.

Exosomes utilized for providing the antigens used in the presently disclosed methods can be isolated from exosome-producing cells. In some embodiments, the cell is a cultured cell, that is, a cell propagated ex vivo in culture media. The cultured cell can be, but is not necessarily, immortalized to facilitate continuous propagation. In some embodiments, the cell is a cancer cell, such as for example a cancer cell originally isolated from a tumor and then propagated in culture, as is generally known in the art. In some embodiments, the cancer cell can be an epithelial cancer or an adenocarcinoma. For example, in some embodiments, the cancer cell can be an ovarian cancer cell, a cervical cancer cell, a breast cancer cell, an endometrial cancer cell, a colon cancer cell, a prostate cancer cell, a lung cancer cell, a melanoma cell, a pancreatic cancer cell, or a choriocarcinoma cell. In some embodiments, the cell is a primary culture cell, such as for example a placental cell isolated from a subject.

In particular embodiments, the cell is a cultured cell line selected from the group including but not limited to a UL-1 cell, UL-2 cell, a UL-3 cell, and UL-6. All of these primary human ovarian tumor cell lines were established in the inventors' laboratories, from women with Stage IIIc cyst adenocarcinoma of the ovary (designated UL-1, UL-2, UL-3, and UL-6). UL-2 and UL-3 were derived from hereditary ovarian cancer, while UL-1 and UL-6 were derived from spontaneous cancers. UL-1 cells were derived from a 63 year old female, UL-2 cells were derived from a 34 year old female, U L-3 cells were derived from a 42 year old female, and UL-6 cells were derived from a 72 year old female patient. These cell lines are tumorigenic in nude mice and give rise to tumors in nude mice that are consistent with cyst adenocarcinomas. These cell lines are all positive for EpCAM, PLAP, FasL, PD-L1 and class II MHC.

In some embodiments, the antigens can be isolated for use in the methods disclosed herein by harvesting a media in which the cells are cultured and selectively removing the exosomes from the media, such as for example by centrifugation. The antigens can then be further isolated from the antigens if desired by routine methods of protein isolation and purification, as is generally known in the art.

Further with respect to the diagnostic methods of the presently disclosed subject matter, a preferred subject is a vertebrate subject. A preferred vertebrate is warm-blooded; a preferred warm-blooded vertebrate is a mammal.

A preferred mammal is most preferably a human. As used herein, the term "subject" includes both human and animal subjects. Thus, veterinary therapeutic uses are provided in accordance with the presently disclosed subject matter.

As such, the presently disclosed subject matter provides for the treatment of mammals such as humans, as well as those mammals of importance due to being endangered, such as Siberian tigers; of economic importance, such as animals raised on farms for consumption by humans; and/or animals of social importance to humans, such as animals kept as pets or in zoos. Examples of such animals include but are not limited to: carnivores such as cats and dogs; swine, including pigs, hogs, and wild boars; ruminants and/or ungulates such as cattle, oxen, sheep, giraffes, deer, goats, bison, and camels; and horses. Also provided is the treatment of birds, including the treatment of those kinds of birds that are endangered and/or kept in zoos, as well as fowl, and more particularly domesticated fowl, i.e., poultry, such as turkeys, chickens, ducks, geese, guinea fowl, and the like, as they are also of economic importance to humans. Thus, also provided is the treatment of livestock, including, but not limited to, domesticated swine, ruminants, ungulates, horses (including race horses), poultry, and the like.

### II. Methods for Diagnosing Cancers

The presently-disclosed subject matter further provides methods for diagnosing and characterizing cancers in subjects. Cancer is the second leading cause of death in the United States. In 1999 there were an estimated 563,100 cancer deaths and each year about 1,222,000 new cancer cases are diagnosed. Among these, solid tumor cancers such as lung, breast, prostate and colorectal cancers are the most common. Cancer diagnosis and classification relies on the subjective interpretation of both clinical and histopathological information by eye with the aim of classifying tumors in generally accepted categories based on the tissue of origin of the tumor. However, clinical information can be incomplete or misleading.

Current diagnostic assays for cancer are most commonly antigen-based. These assays rely on the expression, synthesis, and release of specific proteins by tumor cells either by active secretion or shedding or as a consequence of cell death (either necrosis or apoptosis). These antigenic proteins must "escape" the primary site, saturate the antigen-processing capacity of the individual's immune components, gain access to the circulation, and reach a sufficient steady-state concentration to be detected by enzyme- or radiolabel-based immunoassays. These events occur well after the initial neoplastic transformation event and tumor foci development.

Improvements to detect cancers earlier have primarily focused on enhanced sensitivity and high throughput capacity; however, these diagnostic assays exhibit several fundamental limitations. Circulating antigens do not appear until well after the establishment of the tumor, current antigens are not specific for cancer, and no single antigen is expressed in 100% of cancer cases.

Recently, analysis of proteomic patterns of patients' sera as an early detection method has been investigated (Liotta et al., Gynecol Oncol 88: S25-S28, 2003.). Unfortunately, this mass spectrometry (MS) based-approach has several drawbacks. In addition to the delayed appearance of circulating antigens associated with antigen-based assay systems, MS proteomic analysis has medium sensitivity with diminishing yields with higher molecular weight proteins, it does not identify these marker proteins and it necessitates the use of sophisticated analytical devices, both SELDI-TOF mass spectrometry and bioinformatic tools. In addition to cancer associated protein differences, serum proteomic patterns can exhibit individual variability and results of this "MS-diagnostic fingerprinting" are dependent upon comparison with a "training set" of sera and subsequent interpretation of the resulting patterns. Thus, there are feasibility, reproducibility, and standardization issues that need to be addressed before MS proteomic analysis can be applied clinically.

Evaluation of the immunocompetence of patients with cancer has been utilized to determine whether the tumor has compromised host immunity, to identify specific immune parameters that have prognostic value and to provide a baseline for assessment of immunotherapy (Hellstrom et al., In: DeVita VT, Hellman S, and Rosenberg SA, eds. Biologic therapy of cancer. New York: Lippincott, 1991: 35-52.). While immune cell functions are impaired in many cancer patients, including for example ovarian cancers, as defined by the failure to eradicate the tumor, studies suggest immune recognition of tumor antigens remains intact. Tumor reactive autoantibodies can be detected early in the development and progression of tumors. Studies have indicated the presence of tumor-reactive immunoglobulins in cancer patients, including those with melanoma (Merimsky et al., Tumour Biol 15:188-202,1994), lung (Niklinska et al., Folia Histochem Cytobiol 39:51-56, 2001; Brichory et al., Cancer Res 61:7908-7912, 2001), breast (Conroy et al., Lancet 345:126,1995; Barbouche et al., Europ J Clin Chem Clin Biochem 32: 511-514, 1994), head and neck (Vlock et al, Cancer Res 49:1361-1365,1989) and ovarian cancers (Kutteh et al., J Soc Gynecol Invest 3:216-222,1996; Taylor et al., Am J Reprod Immunol 6:179-184, 1984; Vogl et al., Brit J Cancer 83:1338-1343, 2000). The mechanisms underlying the induction of a humoral response appears to be multifaceted in cancer patients, including point mutations resulting in an altered amino acid sequence (Jung and Schluesener, J Exp Med 173: 273-276,1991; Winter et al., Cancer Res 52: 4168-4174, 1992; Lubin et al., Cancer Res 53:5872-5876,1993), overexpression resulting from amplification or increased protein stability (Peoples et al., Proc Natl Amer Sci USA 92: 432-436, 1995; Labrecque et al., Cancer Res 53:3468-3471, 1993), altered post-translational modifications (Kotera et al., Humoral immunity against a tandem repeat epitope of human mucin muc-1 in sera from breast, pancreatic and colon cancer patients, Cancer Research. 54(11):2856-60, 9994*;* Andersson E. Henderikx P. Krambovitis E. Hoogenboom HR. Borrebaeck CA. A tandem repeat of MUC1 core protein induces a weak in vitro immune response in human B cells. Cancer Immunology, Immunotherapy. 47(5):249-56, 1999 *;* Chinni et al., Clin Cancer Res 3: 1557-1564, 1997), or errors in processing. The extracellular appearance of intracellular proteins commonly results in the generation of autoantibodies. Autoantibodies against aberrant cellular antigens, such as c-myb, c-myc, p53, and p21 ras have been found in a significant proportion of cancer patients, even in the absence of detectable circulating antigen (Canevari et al., Annals Oncol 7:227-232, 1996; Yamamoto et al., Oncology 56:129-133, 1999; Abu-Shakra et al., Annals Rheum Dis 60:433-440, 2001.). The detection of antibodies against these intracellular proteins at the time of diagnosis appears to be associated with a poor prognosis. In previous studies analyzing the induction of autologous antibodies to p53, autoimmune responses associated with protein overexpression tends to be directed at the amino- and carboxy-termini. In contrast, autoantibodies against a related protein, p73, appear to be directed at the interior mutational hot spots. In experimental animal studies using either chemically induced or transplantable spontaneous tumors, circulating tumor reactive IgG can be demonstrated well in advance of palpable tumor or circulating tumor antigen.

The present inventors and colleagues previously developed "autologous typing" to identify the presence of tumor-reactive IgG and to define antigenic recognition patterns of cancer patients as a diagnostic tool (Taylor and Doellgast, Analytical Biochemistry, 98:53-59, 1979.). However, until recently, the molecular identification of the specific antigens eliciting this humoral response remained elusive.

A recent modification of "autologous typing" is currently being used to analyze tumor antigens. The modification, termed SEREX, is the identification of targets of immune recognition using serological analysis of recombinant cDNA expression libraries of human tumors (Old, J Exp Med 187:1163-1167, 1998). This technique possesses several limitations, including high cross-reactivity with bacterial or phage components, the co-expression of cDNA derived from normal tissue (including lymphoid cells) present within the original tumor and, since the cDNA is expressed in a bacterial system, there is an absence of cancer-linked post-translational modifications and processing that occurs within the tumor cell, which can result in the loss of immunoreactivity of these "engineered" protein targets.

Serologic analysis techniques have been used to define new target antigens for cancer diagnosis, but there are deficiencies that limit their utility. Current antigenic targets used to detect autoantibodies are recombinant wild-type proteins - for example, the detection of anti-p53 autoantibodies has exclusively used wild-type p53 protein. The use of wild-type proteins eliminates the detection of autoantibodies against mutational hotspots. In the case of p53, most studies suggest autoantibodies bind to the amino-or carboxy-termini. The failure of recombinant wild-type p53 to reactive with autoantibodies directed against mutated sites may explain the lower frequency of p53 autoantibodies versus the frequency of p53 mutations in ovarian cancer.

The presently-disclosed subject matter provides a novel approach to diagnosis of cancers that addresses the limitations of prior techniques discussed above. The novel application oftumor-reactive humoral responses of cancer patients in protein arrays disclosed herein, using "natural" tumor cell-derived protein antigens (e.g., derived from cancer cell exosomes), provides a innovative and rapid approach to identify the appearance of alterations (e.g., mutations, truncations, and post-translational modifications) linked with the onset and progression of cancer in subjects. Patients with malignant diseases develop autoimmune-like phenomena as a result of generation of autoantibodies against various autoantigens, including oncoproteins, tumor suppressor genes, proliferation associated antigens and cancer/testis antigens. Aberrations in specific proteins that are shared by patients with the same tumor type represent essential neoplastic pathways and these shared alterations can be utilized for the diagnosis and characterization of cancers, including determination of tumor type and stage.

As such, the presently-disclosed subject matter provides in some embodiments a method of diagnosing a cancer in a subject. In some embodiments, the method comprises providing a biological sample comprising or suspected of comprising autoantibodies from a subject; contacting an antigen with the sample, wherein the antigen comprises an autoantibody immunoreactive peptide; detecting the autoantibodies in the sample immunoreactive to the antigen; and comparing a level of autoantibody immunoreactivity to the antigen with a reference level to diagnose the cancer in the subject.

Further, in some embodiments of the presently disclosed subject matter, a method for characterizing a cancer associated with autoantibody production in a subject is provided. For example, the cancer can be further characterized by detecting and/or quantitating autoantibodies in a sample from the subject, such as by determining a stage of the cancer based on a quantitative measure of the level of particular autoantibodies present in the sample. In some embodiments, the method comprises providing a biological sample comprising autoantibodies from a subject; contacting an antigen with the sample, wherein the antigen comprises an autoantibody immunoreactive peptide; detecting the autoantibodies in the sample immunoreactive to the antigen; and quantitating a level of autoantibody immunoreactivity to the antigen to thereby characterize the cancer in the subject. The Examples provide additional details of exemplary embodiments for characterizing the stage of cancer present in a subject.

In some embodiments of the methods for diagnosing and/or characterizing cancer in a subject, the autoantibody immunoreactive peptide can be isolated from an exosomes.

The term "cancer" as used herein refers to all types of cancer or neoplasm or malignant tumors found in animals, including leukemias, carcinomas and sarcomas. Examples of cancers are cancer of the brain, bladder, breast, cervix, colon, head and neck, kidney, lung, non-small cell lung, melanoma, mesothelioma, ovary, prostate, sarcoma, stomach, uterus and Medulloblastoma.

By "leukemia" is meant broadly progressive, malignant diseases of the blood-forming organs and is generally characterized by a distorted proliferation and development of leukocytes and their precursors in the blood and bone marrow. Leukemia diseases include, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

The term "carcinoma" refers to a malignant new growth made up of epithelial cells tending to infiltrate the surrounding tissues and give rise to metastases. Exemplary carcinomas include, for example, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiennoid carcinoma, carcinoma epitheliale adenoides, exophytic carcinoma, carcinoma ex ulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypemephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, naspharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tuberous carcinoma, verrucous carcinoma, and carcinoma villosum.

The term "sarcoma" generally refers to a tumor which is made up of a substance like the embryonic connective tissue and is generally composed of closely packed cells embedded in a fibrillar or homogeneous substance. Sarcomas include, for example, chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilns' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

The term "melanoma" is taken to mean a tumor arising from the melanocytic system of the skin and other organs. Melanomas include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma subungal melanoma, and superficial spreading melanoma.

Additional cancers include, for example, Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, premalignant skin lesions, testicular cancer, lymphomas, thyroid cancer, neuroblastoma, esophageal cancer, genitourinary tract cancer, malignant hypercalcemia, cervical cancer, endometrial cancer, and adrenal cortical cancer.

In some embodiments, the cancer is an epithelial cancer or an adenocarcinoma. For example, in some embodiments, the disorder can be ovarian cancer or cervical cancer that originated from epithelial tissues. As another example, in some embodiments, the disorder can be an adenocarcinoma selected from the group including but not limited to ovarian cancer, cervical cancer, breast cancer, endometrial cancer, colon cancer, prostate cancer, lung cancer, melanoma, and pancreatic cancer.

In some embodiments, one or more antigens can be selected for use in detecting autoantibodies that have been correlated with cancer (and in some embodiments, a particular stage of cancer). Non-limiting examples of antigens to which autoantibodies associated with cancers immunoreact and which can be used with the present methods are listed in the table below.

As one example, cancer antigen peptides such as members of the inhibitor-of-apoptosis proteins (IAP) family (e.g., survivin) can be utilized. Survivin is expressed in the majority of pancreatic adenocarcinomas (Sarela et al. Expression of survivin, a novel inhibitor of apoptosis and cell cycle regulatory protein, in pancreatic adenocarcinoma. Br J Cancer. 2002 Mar 18;86(6):886-92).

Additional antigens useful with the present methods include mucins (e.g., CA125 (MUC-16), TAG-72, and MUC-1). Increased mucin production occurs in many adenocarcinomas, including cancer of the pancreas, lung, breast, ovary, colon, and others. The tumor-associated antigen MUC-1 is overexpressed and underglycosylated in human adenocarcinomas of diverse origins, such as breast, ovary, and colon (Henderikx et al. Human single-chain Fv antibodies to MUC-1 core peptide selected from phage display libraries recognize unique epitopes and predominantly bind adenocarcinoma. Cancer Res. 1998 Oct 1;58(19):4324-32). In addition, the tumor-associated glycoprotein TAG-72 is expressed in the majority of human adenocarcinomas but is rarely expressed in most normal tissues (Yoon et al. Construction, affinity maturation, and biological characterization of an anti-tumor-associated glycoprotein-72 humanized antibody. J Biol Chem. 2006 Mar 17;281(11):6985-92).

Other antigens useful with the present methods include cancer-testis antigens (e.g., NY-ESO-1, SSX-1, SSX-2, SSX-4, and SCP-1). As one example, it has been demonstrated that cancer-testis antigen expression in ovarian serous neoplasms can correlate directly with their degree of malignancy, for example.

Additional antigens useful with the present methods include heat shock proteins (e.g., HSP27, HSP60, HSP90, and GRP78). For example, it has been demonstrated that the expression of HSPs is higher in lung adenocarcinomas. Further, both HSP70 and HSP90 may have relation to the genesis and prognosis of endometrial carcinoma. See also, e.g., Croute et al. Expression of stress-related genes in a cadmium-resistant A549 human cell line. Journal of Toxicology & Environmental Health Part A. 68(9):703-18, 2005; Liang et al. Mislocalization of membrane proteins associated with multidrug resistance in cisplatin-resistant cancer cell lines. Cancer Research. 63(18):5909-16, 2003; Lee. GRP78 induction in cancer: therapeutic and prognostic implications. Cancer Research. 67(8):3496-9, 2007; and Lee et al. GRP78 as a novel predictor of responsiveness to chemotherapy in breast cancer. Cancer Research. 66(16):7849-53, 2006.

Another example of antigens useful with the present methods include mesothelin, which is a differentiation antigen present on normal mesothelial cells and overexpressed in several human tumors, including mesothelioma and ovarian and pancreatic adenocarcinoma (Hassan et al. Mesothelin: a new target for immunotherapy. Clin Cancer Res. 2004 Jun 15;10(12 Pt 1):3937-42; Baruch et al. Immunocytochemical study of the expression of mesothelin in fine-needle aspiration biopsy specimens of pancreatic adenocarcinoma. Diagnostic Cytopathology. 35(3):143-7, 2007; Pu et al. Utility of WT-1, p63, MOC31, mesothelin, and cytokeratin (K903 and CK5/6) immunostains in differentiating adenocarcinoma, squamous cell carcinoma, and malignant mesothelioma in effusions. Diagnostic Cytopathology. 36(1):20-5,2008; Argani et al. Mesothelin is overexpressed in the vast majority of ductal adenocarcinomas of the pancreas: identification of a new pancreatic cancer marker by serial analysis of gene expression (SAGE). Clinical Cancer Research. 7(12):3862-8, 2001; and Dennis et al. Markers of adenocarcinoma characteristic of the site of origin: development of a diagnostic algorithm. Clinical Cancer Research. 11 (10):3766-72, 2005).

| Genus of Ag | Antigen marker | AKA | | Normal cellular location |
|---|---|---|---|---|
| Tumor Suppressor Gene Family | P53 | NY-CO-13 | | nucleus |
| | p63 | | | nucleus |
| | p73 | | | nucleus |
| Nucleic Acid Binding Proteins | B23 | nucleophosmin | | nucleolus |
| | C23 | nucleolin | | nucleolus |
| Anti-Apoptotic Protein | Survivin | | | cytoplasm |
| Oncogene family | c-myc | | | nucleus |
| | c-ras | | | cytoplasm |
| | c-erb2 | HER-2, neu | | plasma membrane |
| | mdm2 | | | nucleus |
| Homeobox Proteins | Hox-A7 | | | nucleus |
| | Hox-B7 | | | cytoplasm |
| Cancer testis Ags | SCP-1 | | | nucleus |
| | SSX-1 | synovial protein 1 | complex | nucleus |
| | SSX-2 | synovial protein 2 | complex | nucleus |
| | SSX-4 | synovial protein 4 | complex | nucleus |
| | NY-ESO-1 | | | cytoplasm |
| Heat Shock Family Proteins | HSP 27 | | | cytoplasm |
| | HSP-60 | | | cytoplasm & nucleus |
| | HSP-90 | | | cytoplasm |
| | GRP-78 | | | cytoplasm |
| Enzyme precursor of Pro-lysosomal enzyme family | proCathepsin D | | | cytoplasm |
| | PLAP | | | plasma membrane |
| Adhesion Molecule | EpCAM | | | plasma membrane |
| Mucins | TAG-72 | | | cytoplasm & plasma membrane |
| | CA125 | | | Plasma membrane |
| | Muc-1 | Mel-CAM | | plasma membrane |
| | Muc-16 | | | plasma membrane |
| Adhesion related protein (glycoprotein) | Mesothelin | | | plasma membrane |
| Kinase Family | EGF kinase | | | cytoplasm |

### III. Methods for Diagnosing Infertility Disorders

The presently-disclosed subject matter further provides methods for diagnosing and characterizing infertility disorders in subjects. Infertility disorders are a common medical condition, affecting approximately 7.3 million Americans every year. It is estimated that about 10% to 15% of married couples who try to conceive are unable to do so after one year. Infertility disorders include female infertility, which is a term health care providers use for women who are unable to get pregnant after at least one year of trying or for those are able to get pregnant but who cannot carry a pregnancy to term. Most cases of female infertility result from problems with ovulation. Some infertility disorders affecting fecundity and fertility include premature ovarian failure (POF), in which the ovaries stop functioning before natural menopause, polycystic ovary syndrome (PCOS), in which the ovaries may not release an egg regularly or may not release a viable, healthy egg and reproductive pathologies resultant from endometriosis. Other infertility disorders include endometriosis, preeclampsia, preterm birth, intrauterine growth restriction, and recurrent pregnancy loss.

At present, health care professionals diagnose infertility by doing a workup which consists of evaluating ovulation status and an exam to identify pathologies of the uterus or fallopian tubes or other causative factors. In many cases, clinical diagnosis is achieved via elimination of common underlying etiologies and after two menstrual cycles. Reproductive failure, including recurrent spontaneous abortion and other infertility disorders, can result from multiple causes.

Autoimmune mechanisms are involved in infertility disorders, such as endometriosis and ovarian failure, and may be responsible for the pathophysiology of pre-eclampsia or spontaneous abortions. Anti-ovarian autoantibodies have been detected in 33-61 % of patients with unexplained infertility, suggesting that this pathology may represent an early stage of autoimmune ovarian failure. As in other autoimmune pathologies (such as type 1 diabetes mellitus and thyroiditis), antiovarian antibodies may appear months or years before the onset of clinical symptoms, thus they could predict future ovarian failure in women with unexplained infertility.

Endometriosis is a disease that afflicts up to 10% of reproductive-age women and is characterized by hormone-regulated growth of endometrial tissue outside the uterus. Endometriosis is known to be a cause of female infertility disorders in 30-50% of affected women. It has been suggested that autoimmune mechanisms may be involved, and antibodies against different candidate autoantigens have been demonstrated in these patients. While the mechanism of infertility in endometriosis is not well understood, endometriosis has been shown to be associated with autoantibodies and/or other autoimmune diseases in up to two-thirds of patients. It has been reported that the presence of anti-endometrial antibodies in 100% and anti-ovarian antibodies in 62% of patients with endometriosis.

The involvement of autoimmunity has also been studied in POF with the overall proportion of autoimmune forms of POF being estimated between 20-70%. The human ovary can be the target of an autoimmune attack in various circumstances, including several organ-specific or systemic autoimmune diseases. Clinically, the ensuing ovarian dysfunction often results in POF, but other pathologies involving the ovaries, such as unexplained infertility, PCOS and endometriosis have been associated with anti-ovarian autoimmunity. The diagnosis of an autoimmune mechanism in these pathologies has relied on the detection of anti-ovarian autoantibodies, but recently special attention has also been focused on the cellular component of the autoimmune response. However, little is known about the molecular targets of the autoimmune effectors, and very few autoantigens have been formally identified.

The detection of autoantibodies directed against various ovarian targets supports the hypothesis of an autoimmune etiology of POF. The initial reports on anti-ovarian antibodies included mainly patients with POF and an associated adrenal autoimmune disease. These patients had antibodies that recognized several types of steroid-producing cells of the adrenal cortex, testis, placenta and ovary and were termed steroid cell antibodies (SCA). The prevalence of SCA was dependent on the clinical features: they can be detected in approximately 60% of APS-I patients and 25-40% of APS-II patients, but the highest prevalence (78-100%) has been shown in patients with POF. It has also been shown that 33-43% of normally cycling women with polyendocrinopathy and SCA would develop ovarian failure within 8-15 years.

Another common cause of reproductive failure is PCOS which is characterized by a chronic hyperandrogenic anovulatory state associated with a number of clinical symptoms and affects 5-10% of women of reproductive age. Although PCOS, as well as polycystic ovaries without the syndrome, are related to hormonal dysregulation, autoimmune disturbances have been demonstrated.

Histopathological features of autoimmune oophoritis with a cystic aspect associated with anti-ovarian serum antibodies have been reported. Several investigators have addressed the prevalence of organ-non-specific and organ-specific autoantibodies and have demonstrated anti-ovarian antibodies in 50-60% of PCOS patients. Tung reported the production of oocyte autoantibodies in a murine model resulting in ovarian failure. Autoimmune dysfunction in clinically asymptomatic patients also may lead to recurrent spontaneous abortions. Some recurrent aborters have, in fact, one or more types of abnormal autoantibodies. Antiphospholipid antibodies, anti-DNA antibodies, and antinuclear antibodies have been implicated in recurrent abortions. One mechanism in spontaneous abortion is thought to be thrombosis of the placental vasculature and placental infarction, caused by the reaction of autoantibodies against β2-glycoprotein I, prothrombin, and/or annexin V. Another possible cause is direct binding of autoantibodies to cytotrophoblast cells, impairing trophoblast invasion into maternal decidua and implantation by preventing differentiation to syncytiotrophoblast.

The immune system has been shown to play a significant role in the pathogenesis of premature ovarian failure, polycystic ovarian syndrome, endometriosis, recurrent pregnancy loss, and other infertility disorders. The presently-disclosed subject matter provides for the use of markers of immunoreactivity (e.g., autoantibodies associated with an infertility disorder) as a diagnostic aid for infertility disorders, including but not limited to POF, PCOS, endometriosis, preeclampsia, preterm birth, intrauterine growth restriction, and recurrent pregnancy loss. Further, the evaluation of autoimmunity against specific components on the reproductive tract, as provided by the presently-disclosed subject matter, is a tool of prognosis for infertility treatments.

As such, in some embodiments of the presently disclosed subject matter, a method of diagnosing an infertility disorder in a subject is provided. In some embodiments, the method comprises providing a biological sample comprising or suspected of comprising autoantibodies from a subject; contacting an antigen with the sample, wherein the antigen comprises an autoantibody immunoreactive peptide; detecting the autoantibodies in the sample immunoreactive to the antigen; and comparing a level of autoantibody immunoreactivity to the antigen with a reference level to diagnose the infertility disorder in the subject.

Further, in some embodiments of the presently disclosed subject matter, a method for characterizing an infertility disorder associated with autoantibody production in a subject is provided. In some embodiments, the method comprises providing a biological sample comprising autoantibodies from a subject; contacting an antigen with the sample, wherein the antigen comprises an autoantibody immunoreactive peptide; detecting the autoantibodies in the sample immunoreactive to the antigen; and quantitating a level of autoantibody immunoreactivity to the antigen to thereby characterize the infertility disorder in the subject.

For example, in some embodiments the infertility disorder is a disorder selected from the group including but not limited to premature ovarian failure (POF), polycystic ovary syndrome (PCOS), endometriosis, preeclampsia, preterm birth, intrauterine growth restriction and recurrent pregnancy loss (spontaneous abortion). Further, in some embodiments, the detected autoantibodies are immunoreactive to antigens derived from ovary, endometrium, placenta, or combinations thereof.

In some embodiments of the methods for diagnosing and/or characterizing infertility disorders in a subject, the autoantibody immunoreactive peptide can be isolated from an exosome. In some embodiments, the autoantibody immunoreactive peptide is a peptide antigen selected from the group consisting of: nuclear antigens with molecular weights of about 50kD and 80kD and membrane antigens with molecular weights of about 10kD, 30kD, 45kD, 90kD and 125kD. Recognition of membrane proteins is shared by all infertilities; however, nuclear protein recognition appears to be unique to endometriosis.

### IV. Kits for Detecting Autoantibodies

In further embodiments, the presently disclosed subject matter provides immunological kits for use in detecting autoantibodies in biological samples. Such kits can generally comprise one or more antigens disclosed herein that can immunoreact with the tested for autoantibodies. In some embodiments, the antigens are isolated from exosomes, as disclosed herein. More specifically, the immunodetection kits will thus comprise, in suitable container(s), one or more autoantibody immunoreactive peptide antigens. In some embodiments, the kits further comprise antibodies that bind to the antigens and/or antibodies that bind to other antibodies (e.g., autoantibodies of interest) via, for example, Fc portions.

In certain embodiments, the antigen or can be provided bound to a solid support, such as for example a column matrix or well of a microtiter plate, a membrane (e.g., nitrocellulose, PVDF or similar material), beads, or dipsticks. Alternatively, the support can be provided as a separate element of the kit.

The immunodetection reagents of the kit can include detectable labels that are associated with, or linked to, the given detecting antibody or to the antigen itself. Detectable labels that are associated with or attached to a secondary binding ligand are also contemplated. Such detectable labels include dyes, haptens, chemiluminescent or fluorescent molecules (rhodamine, fluorescein, green fluorescent protein, luciferase), biotin, radiolabels (³H, ³⁵S, ³²p, ¹⁴C_{,} ¹³¹I) or enzymes (alkaline phosphatase, horseradish peroxidase).

The kits can further comprise suitable standards of predetermined amounts, including both antibodies and antigens. These can be used to prepare a standard curve for a detection assay.

The kits of the presently disclosed subject matter, regardless of type, can generally comprise one or more containers into which the biological agents are placed and suitably aliquoted. The components of the kits can be packaged either in aqueous media or in lyophilized form.

The compositions of the presently disclosed subject matter can be advantageously packaged into a kit comprising the active reagent(s), a suitable container, and even instructions for use of the kit. The reagent(s) of the kit can be provided as a liquid solution, attached to a solid support or as a dried powder. When the reagent is provided in a liquid solution, the liquid solution can be an aqueous solution. When the reagent provided is attached to a solid support, the solid support can be chromatograph media, a test plate having a plurality of wells, or a microscope slide. When the reagent provided is a dry powder, the powder can be reconstituted by the addition of a suitable solvent, which may be provided.

The container of the kits can generally include at least one microtiter plate well, slide, vial, test tube, flask, bottle, or even syringe or other container, into which the antigen can be placed, and if desired, suitably aliquoted. Where a second or third binding ligand or additional component is provided, the kit can also generally contain a second, third or other additional container into which this ligand or component can be placed.

The kits of the present subject matter can also typically include a mechanism for containing the antigen(s) container and any other reagent containers in close confinement for commercial sale. Such containers can include injection or blow-molded plastic containers into which the desired containers are retained.

### EXAMPLES

The following Examples have been included to illustrate modes of the presently disclosed subject matter. In light of the present disclosure and the general level of skill in the art, those of skill will appreciate that the following Examples are intended to be exemplary only and that numerous changes, modifications, and alterations can be employed without departing from the scope of the presently disclosed subject matter.

### EXAMPLE 1

### ASSOCIATION OF REACTIVE ANTIBODIES WITH CANCER

Autoreactive antibodies have been ubiquitously demonstrated in all cancer patients tested. The appearance of tumor reactive autoantibodies was analyzed in ovarian cancer patients (n=28) and non-tumor-bearing age-matched female volunteers (n=32). The presence and reactivity of IgG within the sera of ovarian cancer patients with cellular antigens derived from ovarian tumors was quantitated by ELISA.

Tumor-derived cellular antigens from UL-1 ovarian tumor cells, in log phase growth, were diluted 1:25 in a coupling buffer consisting of 100mM sodium carbonate and 0.5M NaCl, pH8.3. Aliquots were added to wells of a 96-well Immulon4 microtiter plate and incubated overnight at 37°C. The plates were blocked with 5% nonfat dried milk and then incubated with 200µl sera, diluted 1/200, from ovarian cancer patients and age-matched normal controls, overnight at 4°C. The plates were washed and incubated with peroxidase-conjugated anti-human IgG (diluted 1/5000) and the presence of bound antibody was determined by incubating the wells with a 50mM citrate buffer solution containing OPD (0.4mg/ml), measuring absorbance at 490nm.

All patients with ovarian cancer exhibited a level of autoantibodies, recognizing ovarian tumor-derived proteins, significantly greater than their non-cancer bearing counterparts (p<0.0001). The level of IgG binding observed in sera from the control population represents a background level. No overlap in IgG binding to tumor antigens was observed between the cancer patient and control groups.

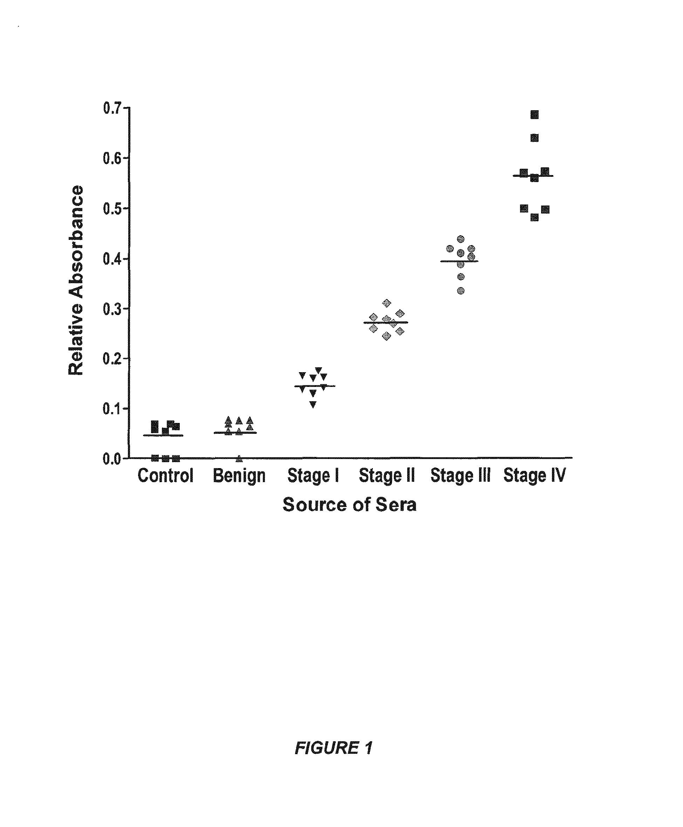

Since cancer patients exhibited an enhanced level of tumor-reactive antibodies and the level of these autoantibodies exhibited significant variability, statistical comparisons were made between the relative absorbance for these tumor-reactive IgG and stage of disease. Data on the level of immunoglobulins reactive with cellular proteins from the UL-1 ovarian tumor cell line were separated by stage of disease (Figure 1). The level of reactive IgG was observed to increase with stage of disease. Sera from stage I cancer patients (n=8) exhibited a significant increase above the background of normal sera (n=32, p<0.001) or sera from women with benign ovarian disease (n=8, p<0.01). No significant difference was observed for immunoreactivity detected in sera from stage I ovarian cancer and ovarian tumors of low malignant potential. The level of immunoreactivity present in stage II patients (n=10) was significantly greater than the level observed in stage I patients (p<0.001) and significantly less than that detected in the sera of stage III patients (n=8, p<0.01). The level of immunoreactivity present in stage III patients was significantly less than the level observed in stage IV patients (n=8, p<0.001). Thus, ovarian cancer patients exhibit tumor-reactive antibodies that correlated with stage of disease.

### EXAMPLE 2

### RECOGNITION OF SPECIFIC ANTIGENIC TARGETS BY TUMOR REACTIVE IgG

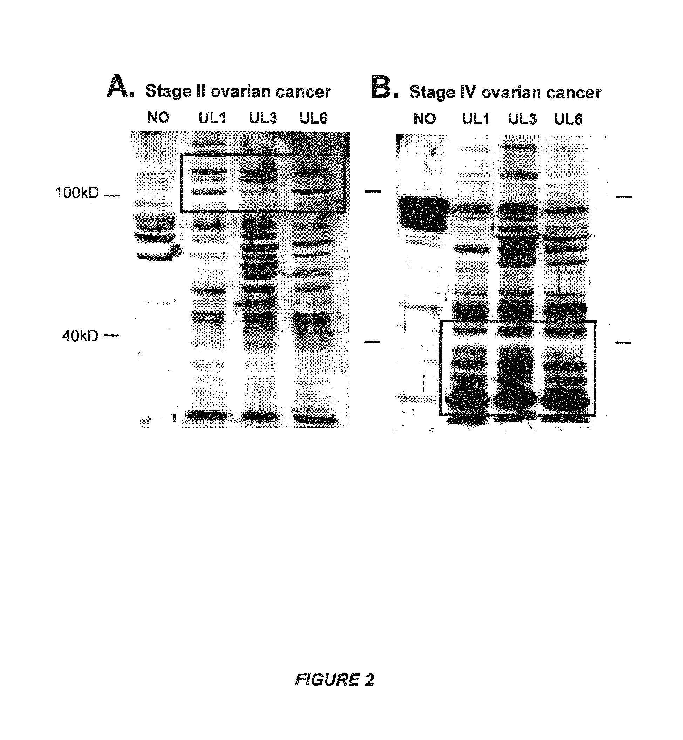

Since quantitative differences in reactivity of autoantibodies obtained from the sera of ovarian cancer patients to ovarian tumor antigens could be demonstrated based on stage of disease, qualitative differences were then investigated using western immunoblotting and densitometry. Using cellular protein antigens from 3 ovarian tumor cell lines (UL-1, UL-3, UL-6) in log phase growth, the presence of components reactive with the humoral immune response of ovarian cancer patients was assessed by western immunoblot (Figure 2, representative blots). Western immunoblotting was performed using patients' sera (diluted 1:100) as the source of primary antibodies. The binding of patient-derived antibodies to tumor cell-derived antigens was visualized using peroxidase-conjugated anti-human IgG, followed by ECL. The immunoreactivity was quantitated by densitometry. Differences in the antigens recognized and the intensity of that recognition were analyzed.

Ten (10) Stage I sera, ten (10) Stage II sera, ten (10) Stage III sera and twelve (12) Stage IV sera were analyzed. For all ovarian cancer patients, these western immunoblots identified multiple bands ranging in molecular weight from 10 to 140kD, although the number and intensity of the immune interaction was variable among patients. The variable intensities of signal on the immunoblot correlated with stage of disease (total pixels per lane, correlation efficient r=0.906).

While, in general, late stage cancer patients recognized more bands at greater intensity, stage-specific differential recognition patterns were observed in the IgG from ovarian cancer patients on these western immunoblots. In addition to stage-related quantitative differences, early stage patients exhibited unique, intense recognition of several antigens with molecular weights greater than 100kD (shown by box in Panel A), while late stage patients exhibited unique recognition of antigens with molecular weights less than 40kD (shown by box in Panel B). As a control, the reactivity of normal sera was also tested against these proteins, as was the reactivity of the cancer patients' sera against normal ovarian epithelium. Sera from normal (non-tumor bearing) female controls failed to recognize proteins by western immunoblot, while cancer patient sera exhibit reactivity with only a few bands in normal ovarian epithelium.

Since some antigens are recognized by early stage sera and continue to be recognized by sera from advanced patients, this demonstrates that these antigenic proteins are expressed early and their expression maintained throughout the tumor's progression. Some proteins appear to only be recognized late in tumor progression, demonstrating their later alteration and/or appearance. However, since these share recognition among patients, they thus represent a common alteration in these patients. Some antigenic proteins appear to be preferentially recognized early, demonstrating the appearance of an altered protein essential from early tumor development. The identification of these stage specific altered proteins provides an additional insight into alterations essential for each stage of tumor development and progression.

The work of other investigators using either western blotting or tumor-derived antigen-based ELISA have detected tumor reactive antibodies in all patients tested: however, assessment of recognition of specific antigenic proteins has not identified a single component in 100% of patients, with most single proteins being recognized in only 10-40% of patients. This limited recognition may be due to most studies on tumor-reactive autoantibodies using recombinant proteins (in many cases, wild-type proteins), which lack cancer-linked post-translational modifications that can modify antigenic epitopes, which can underestimate the actual level of immunoreactivity. Autoantibody responses to antigens broadly expressed in normal and cancer tissues appears to be attributable to tumor-specific mutations or post-translational modifications. While tumor-reactive autoantibodies can be detected in all cancers, tumor-reactive IgG recognizing these specific antigens from non-cancer-bearing volunteers is a rare (<1%) event and in the presently disclosed assay system, they are not detected.

### EXAMPLE 3

### MARKERS IDENTIFYING THERAPEUTIC RESPONSIVENESS

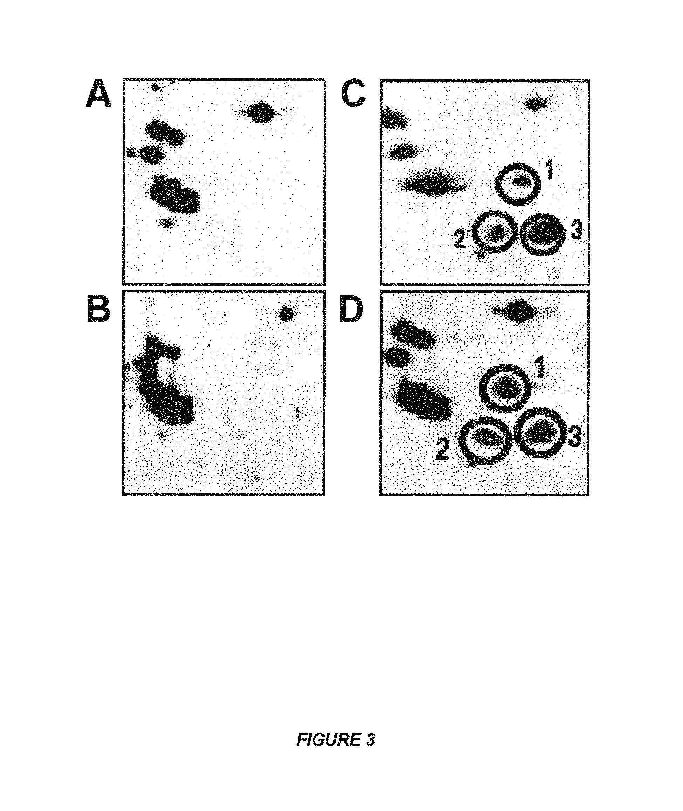

Since differences in reactivity patterns were observed among patients with advanced disease, the correlation between such differences and chemoresistance was examined. Separation of cellular components by 2D electrophoresis followed by western immunoblotting allowed the assessment of some of these differences. Using sera from patients failing to initially respond to cisplatin therapy and patients exhibiting an initial response who remained disease-free for >12 months, recognition of a cluster of 3 spots associated with cisplatin-resistance were identified (Figure 3). Utilizing these cellular antigens as part of the diagnostic array disclosed herein can permit the early identification of resistance tumors.

### EXAMPLE 4

### IDENTIFICATION OF IN VIVO RECOGNIZED ANTIGENIC PROTEINS

Since the patients' humoral responses were directed against specific stage-linked proteins associated with ovarian tumors, the identification of these immunoreactive antigens was initiated by developing a protein array. To minimize the number of total proteins analyzed, immunoreactive proteins were isolated by immunoprecipitation.

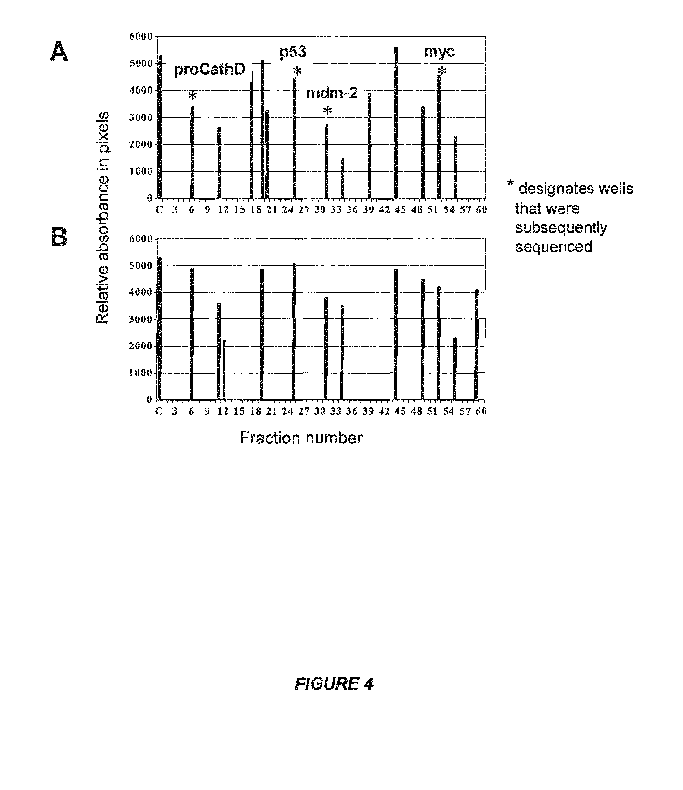

IgG was isolated from sera of 3 women with advanced ovarian cancer, using a 1ml HITRAP PROTEIN G-SEPHAROSE^{®} column (GE Healthcare). The bound IgG fraction was eluted with IMMUNPURE^{®} elution buffer (GE Healthcare), monitoring at 280nm. The IgG-containing fractions were pooled and concentrated and then coupled to HITRAP NHS^{™} columns (GE Healthcare), by the manufacturer's instructions. Cellular proteins from UL-1 ovarian tumor cells were solubilized and clarified. Aliquots of the immobilized patient IgG were incubated with the cellular protein preparations, overnight at 4°C and then the bound complexes were eluted. These immunopurified cellular proteins were fractionated by RP-HPLC chromatography on a 4.6x250mm C8 (300µ) column. The resulting fractions were dried by speed-vac and used to develop a protein array. The protein solution (2.5µl) was manually loaded onto a single spot. A peroxidase-conjugated Ig sample was spotted onto each membrane as a positive control and for orientation of the array (indicated as lane C in Figures 4A and 4B). Sera from advanced stage ovarian cancer patients (diluted 1:100) were incubated with the membranes overnight at 4°C and membranes were then incubated with peroxidase-conjugated anti-human IgG, visualizing by ECL. The resulting film was imaged and analyzed using Kodak analysis software for spot recognition and quantitation.

Each ovarian cancer patient analyzed recognized multiple proteins; however, all patients recognized some proteins. Using this array system, control (non-cancer patient) sera (n=10) failed to recognize any protein targets. To identify the specific proteins responsible for this observed immunoreactive with patients' autoantibodies, portions of the proteins corresponding to those recognized by the humoral immune responses of ovarian cancer patients were subjected to matrix-assisted laser desorption-time of flight (MALDI-TOF) mass spectrometry following trypsin digestion. The resulting peptides were fractionated by HPLC on a fused silica microcapillary column (75x200µm, Polymicro Technologies, Inc.) and eluted directly into the electrospray ion source of a triple quadruple mass spectrometer (TSQ 70, Finnigan MAT) using a linear gradient of 0 to 80% acetonitrile in 0.1M acetic acid (140 B solvent delivery system). Mass spectra were acquired for each peak and the resulting molecular weight fragments for each protein were compared to databases of other known proteins for identity. Additional common peaks (11,19, 44, and 49) were also analyzed; however, these fractions consisted of multiple proteins and sequencing could not be directly pursued.

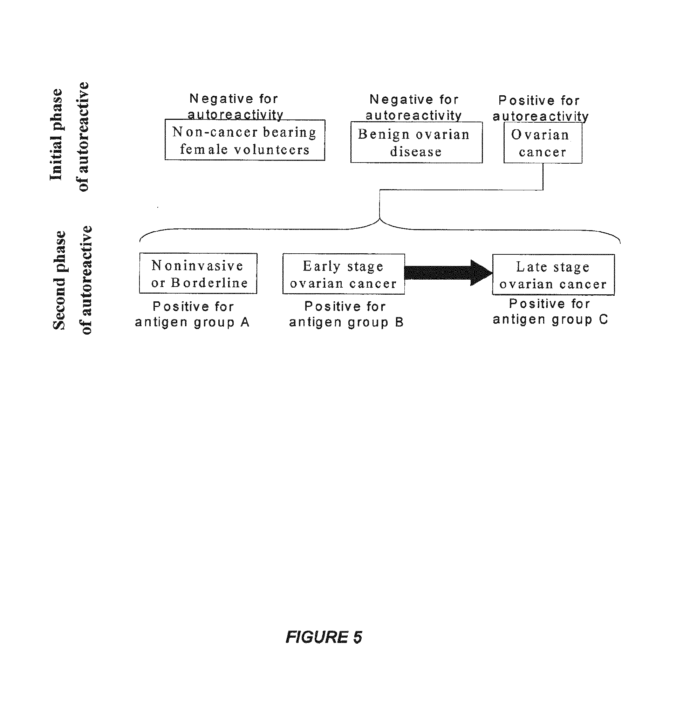

The present methodology can identify a group of antigens recognized only by patients with ovarian cancer (Figure 5). The data disclosed herein indicate the presence of reactive components that correlate with the presence of disease, stage, and chemoresistance. Several groups have proposed that proteins are expressed early in cancer development and that many of these have been shown to elicit autoantibodies. Disis et al. (Global role of the immune system in identifying cancer initiation and limiting disease progression. Journal of Clinical Oncology. 23(35):8923-5, 2005 Dec.) demonstrated that cancer patients mount serum antibody responses to tumor-associated antigens at an early stage of disease. Autoantibodies against p53 have been reported in patients with early stage ovarian, colorectal and oral cancers. The present findings indicate a significant difference in immunoreactivity versus stage, and even recognition in early stage cancer is distinct from normal and benign ovarian disease. These difference represent quantitative differences (binding intensity or titer) rather than qualitative differences; however, the design of the presently-disclosed diagnostic array allows both the identification of autoantibody presence and the quantitation of their binding (intensity). Thus, quantitative differences in tumor-reactive antibody binding (intensity), which we have already demonstrated to exist, provides adequate differentiation of cancer status and stage for diagnosis.

### EXAMPLE 5

### ASSESSMENT OF A PROTEIN ARRAY WITH ESTABLISHED PROTEIN TARGET

Using commercial antibodies against autoantigenic proteins identified previously by the present inventors (Table 1), proteins were isolated from solubilized UL-1 ovarian cancer cells by immunoprecipitation. These isolated proteins were used to establish a protein array to define the efficacy of the present approach. These immunopurified cellular proteins were used to develop a protein array, consisting of 12 spots in a total size of 1.5x2cm. Each of the 12 protein solutions (2.5µl) was manually loaded onto single spots. Peroxidase-conjugated IgG was spotted onto each membrane as a positive control and for orientation of the array.

Sera from normal female controls (n=20), women with benign ovarian disease (n=20) and women with invasive ovarian cancer (n=20) at a 1:100 dilutions were incubated with the membranes overnight at 4°C. The membranes were then incubated with peroxidase-conjugated anti-human IgG and visualized by ECL. The resulting film was imaged and analyzed using Kodak analysis software for spot recognition and quantitation. The absorbance of greater than 900 pixels was set as positive and the percent of sera positive for immunoreactivity was compared.

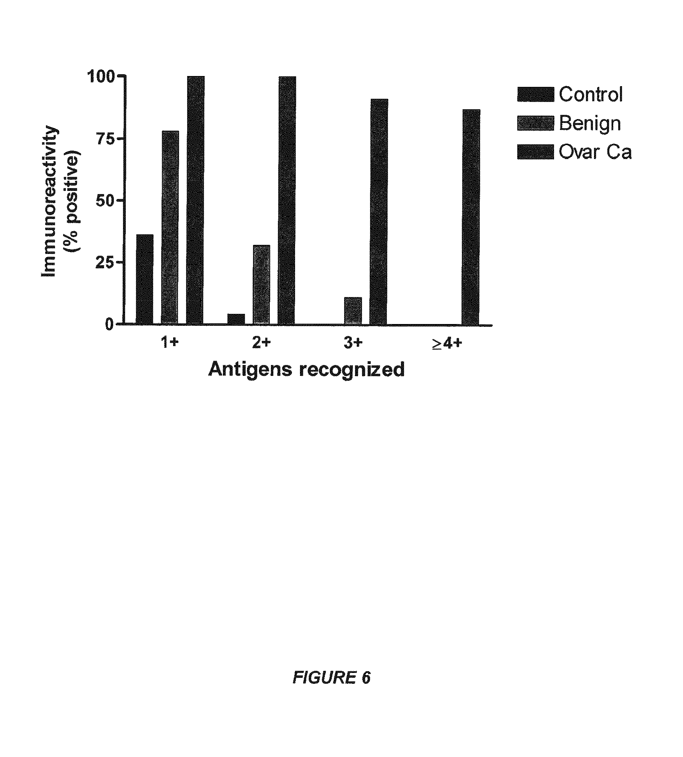

Sera from all women with invasive ovarian cancer recognized at least 2/12 antigens tested; however, sera from controls and women with benign disease also exhibited significant recognition of at least one antigen. In contrast, recognition of four or more of these antigens is limited to sera from women with invasive ovarian cancer. Setting recognition of 4 or more antigens as the cutoff produced an assay with the ability to differentiate between patients with invasive ovarian cancer and benign ovarian disease with a sensitivity = 100%, a specificity = 76% and a positive predictive value = 100%. See Figure 6.

**Table 1: Cellular antigens isolated from ovarian cancer cells used for the array and the percent of ovarian cancer patients with autoantibodies against each protein in the assays.**

| **Antigenic Protein Target** | **Patients with autoreactive IgG (%)** |
|---|---|
| **Homeboxgene family** | |
| Hox A7 | 11/36 (30.6%) |
| Hox B7 | 9/36 (25.0%) |
| **Tumor suppressor gene family** | |
| p53 | 23/36 (63.9%) |
| p63 | 10/36 (27.8%) |
| p73 | 7/36 (19.4%) |
| **Oncogenes** | |
| myc | 14/36 (38.9%) |
| ras | 12/36 (33.3%) |
| **Growth factor related gene family** | |
| c-erb2/HER/neu | 13/36 (36.1 %) |
| A114 (EGFRkinase) | 8/36 (22.2%) |
| **Others** | |
| Placental type alkaline phosphatase | 19/36 (52.8%) |
| NY-ESO-1 | 17/36 (47.2%) |
| EpCAM | 27/36 (75.0%) |

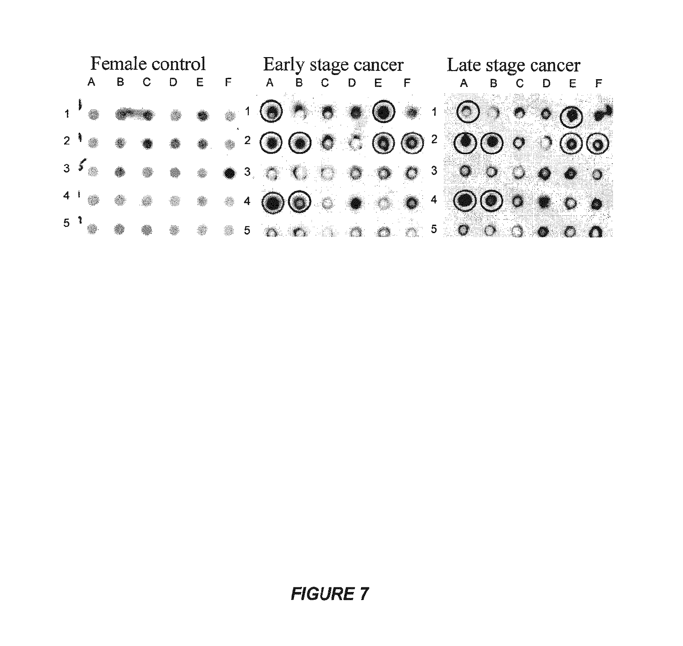

To define additional parameters, beyond differentiating benign and malignant ovarian masses, additional tumor-derived proteins were examined. Figure 7 presents an image from a 36 protein array. Based on these results, this array format can differentiate antigen recognition associated with early versus late stage ovarian cancer. The data presented in Figure 7 is based on antigens from a single ovarian cancer cell line (UL-1). Certain antigens from other tumor cell lines can exhibit more intense reactivity. The optimal antigens from the different ovarian tumor lines, which exhibit maximum cross-reactive among all ovarian cancer patients, can be specifically determined for use in diagnosis of early stage diseases.

### EXAMPLE 6

### ASSESSMENT OF AN ARRAY OF PROTEINS ISOLATED FROM DIFFERENT CANCERS

The table below indicates the layout of an array, indicating the 20 antigens isolated from 4 different ovarian cancers (resulting in a total of 80Ag's) that was assessed for ability to detect autoantibodies in cancer patients as compared to controls. The four ovarian cancer cell lines utilized were A = UL-1, B = UL-2, C = UL-3, and D = UL-6, which are disclosed in detail in the Detailed Description.

**Table 2: Layout of array of antigens for detecting autoantibodies**

| HRP | hulgG | hulgG | hulgG | hulgG | hulgG | BSA | blank |
|---|---|---|---|---|---|---|---|
| p53-A | p53-B | p53-C | p53-D | p63-A | p63-B | p63-C | p63-D |
| p73-A | p73-B | p73-C | p73-D | B23-A | B23-B | B23-C | B23-D |
| C23-A | C23-8 | C23-C | C23-D | CA125-A | CA125-B | CA125-C | CA125-D |
| MUC1-A | MUC1-B | MUC1-C | MUC1-D | MUC16-A | MUC16-B | MUC16-C | MUC16-D |
| cerb//HER-A | cerb//HER-B | cerb//HER-C | cerb//HER-D | NY-ESO1-A | NY-ESO1-B | NY-ESO1-C | NY-ESO1-D |
| SCP1-A | SCP1-B | SCP1-C | SCP1-D | SSX1-A | SSX1-B | SSX1-C | SSX1-D |
| SSX2-A | SSX2-B | SSX2-C | SSX2-D | SSX4-A | SSX4-B | SSX4-C | SSX4-D |
| WSP27-A | HSP27-B | HSP27-C | HSP27-D | HSP60-A | HSP60-B | HSP60-C | HSP60-D |
| GRP78-A | GRP78-B | GRP78-C | GRP78-D | HSP90-A | HSP90-B | HSP90-C | HSP90-D |
| HoxA7-A | HoxA7-B | HoxA7-C | HoxA7-D | HoxB7-A | HoxB7-B | HoxB7-C | HoxB7-D |
| PLAP-A | PLAP-B | PLAP-C | PLAP-D | EpCAM-A | EpCAM-B | EpCAM-C | EpCAM-D |
| ras-A | ras-B | ras-C | ras-D | myc-A | myc-B | myc-C | myc-D |
| procathD-A | procathD-B | procathD-C | procathD-D | mesothelinA | mesothelinB | mesothelinC | mesothelinD |
| survivin-A | survivin-B | survivin-C | survivin-D | EGFK-A | EGFK-B | EGFK-C | EGFK-D |
| mdm2-A | mdm2-B | mdm2-C | mdm2-C | TAG72-A | TAG72-B | TAG72-C | TAG72-D |

For cervical cancer, the serum of each of the following sixteen subjects was evaluated to detect for the presence of antibodies in response to proteins isolated from the cervical cancer cell lines. This included twelve subjects with cervical cancer (adenosquamous, squamous cell and adenocarcinoma), and four controls (subjects without cervical cancer). The reactivity was also evaluated after the cell cultures were treated with retinoic acid.

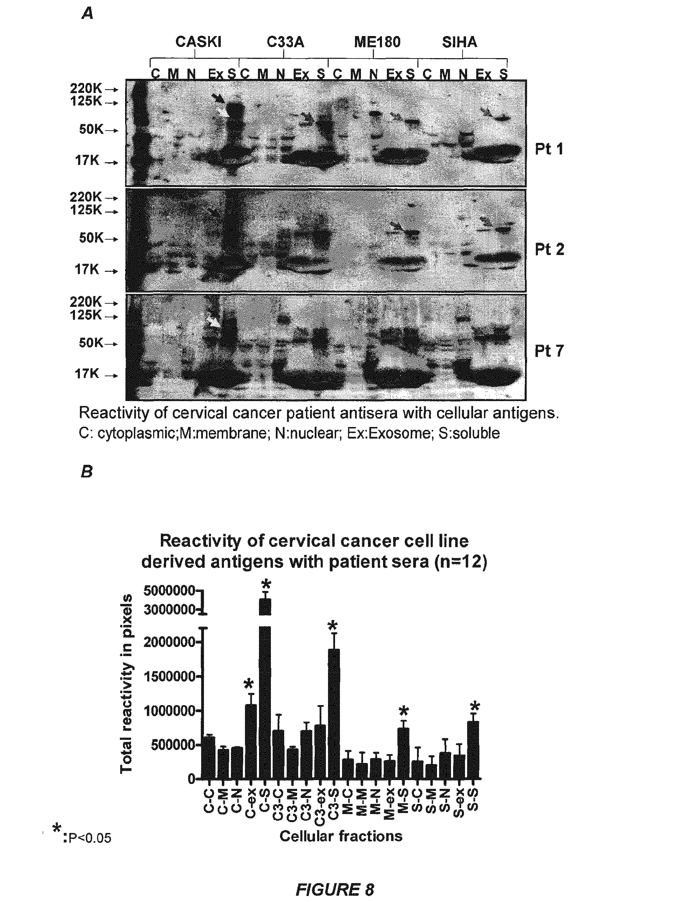

The most reactivity was detected in the soluble fractions of all cell lines (ME180, SiHa, CaSki and C33A) and was statistically significant (p<0.05) (Figures 8A and 8B). The least reactivity was detected in the membrane fraction of all cell lines. The reactivity was quantified in pixels. Overall, CaSki (HPV 16 and 18) demonstrated the most reactivity and when compared to the other cell lines had the most reactivity in the exosomal and soluble fractions (p<0.05). C33A, the cell line without HPV, demonstrated the most reactivity in the membrane fraction. Me180 (HPV-39) demonstrated the least amount of reactivity.

Similar recognition of antigens was detected across each cell line. There was differential recognition of antigens between different cell lines. The control sera did not demonstrate any reactivity.

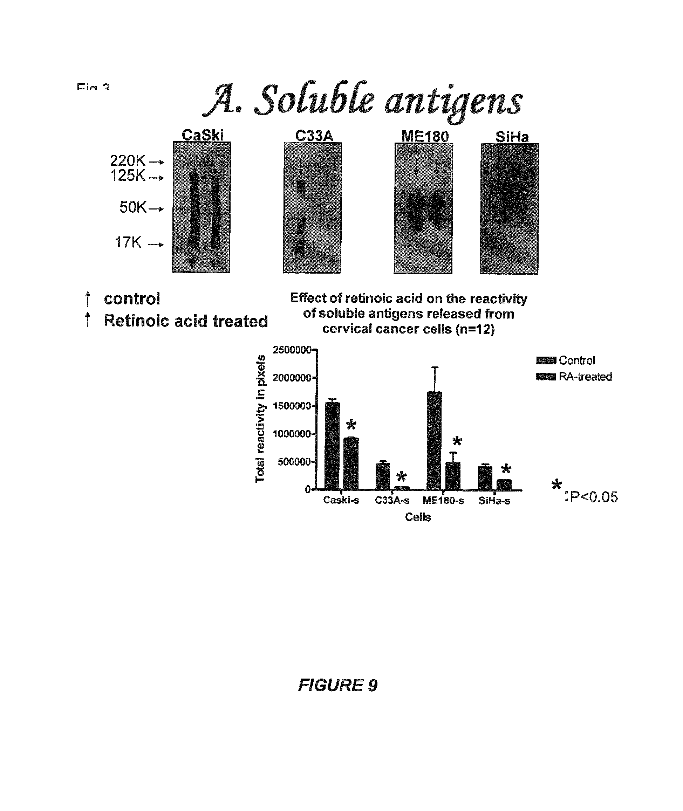

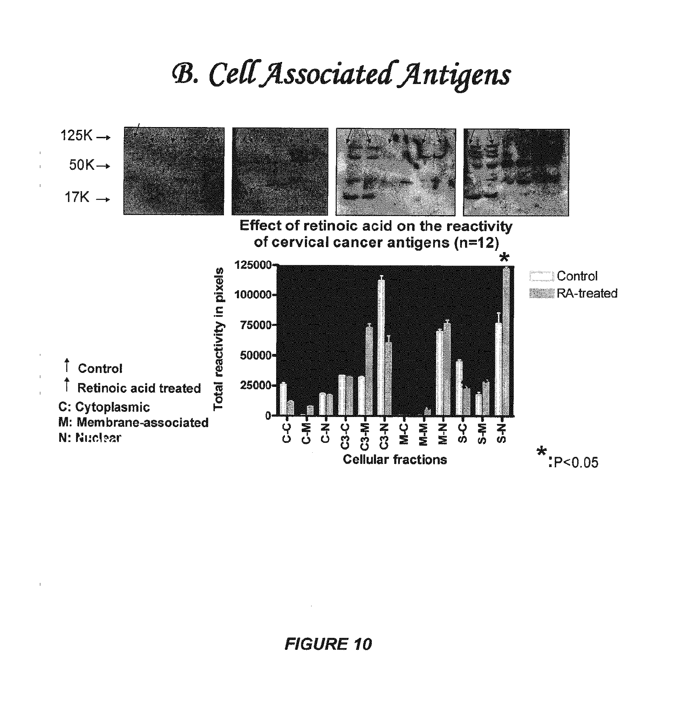

In the sera that were treated with retinoic acid, there was decreased reactivity the soluble cell associated antigens (p<0.05) in all cell lines (Figure 9). Increased reactivity was noted with the cell associated antigens. This was detected in the membrane fraction of all cell lines (p<0.05), the nuclear fraction in C33A and SiHa cell lines (p<0.05), and the cytosolic fraction of the SiHa cell line (p<0.05) (Figure 10).

### EXAMPLE 7

### ASSESSMENT OF DIAGNOSTIC ASSAY ACROSS MULTIPLE DIFFERENT CANCERS

### Methods for Example 7

Recognition of specific antigenic targets by tumor reactive IgG. Since quantitative differences in reactivity of autoantibodies obtained from the sera of ovarian cancer patients to ovarian tumor antigens could be demonstrated based on stage of disease, qualitative differences were then investigated using ELISA immunoblotting to quantify the level of immunoreactivity with specific antigens. Exosomes were isolated from the conditioned media of 3 ovarian tumor cell lines (UL-1, UL-3, UL-6) in log phase growth.

Preparation of specific reactive antigens. Reactive proteins for assay targets were isolated from purified exosomes by immunosorbent chromatography. Commercial antibodies for each protein of interest were obtained. These antibodies were immobilized on 1 ml HITRAP NHS^{™} columns (GE Healthcare), by the manufacturer's instructions. Following exosome centrifugation at 100,000xg, the pellet was solubilized in 50mM Tris-HCl (pH7.5), containing 0.3% SDS, 2mM sodium orthovanadate, 200mM DTT, 1mM sodium fluoride, 1mM sodium pyrophosphate, 1µg/ml leupeptin, 1µg/ml aprotinin, 1µg/ml pepstatin, and 1 mM PMSF on ice. The lysate was sonicated and centrifuged at 10,000xg for 15 minutes. The solubilized proteins were clarified by incubation with Protein G-agarose (Sigma Chemical Co., St. Louis, MO) for 1 hour. This clarified solubilized protein material was applied to the immunosorbent column and incubated overnight at 4°C. The bound material was washed 3 times with PBS containing 1% Triton X-100 and the specific antigenic proteins released by 0.1M glycine-HCl, pH2.8, neutralized with 1M Tris. This antigen preparation was applied to MACROSPHERE GPC^{™} 150/60 columns (4.6x500) (Grace, Deerfield, IL) equilibrated in TBS and run isocratically. Aliquots of the eluates were evaluated by western immunoblot to confirm the appropriate molecular fraction. The appropriate antigen fraction was further separated by RP-HPLC chromatography on a 4.6x250mm C8 (300µ) column. The protein peak was dried by vacuum centrifugation and resuspended in TBS and quantitated by protein assay.

ELISA defined autoantibody reactivity. To define the level of reactivity of patient-derived autoantibodies to tumor-derived exosomal, an ELISA assay was performed. Isolated proteins from each immunosorbent preparation were diluted 1:25 in a coupling buffer consisting of 100mM sodium carbonate and 0.5M NaCl, pH8.3. Aliquots (2µg/well) were added to wells of a 96-well IMMULON 4^{™} microtiter plate (Chantilly, VA) and incubated overnight at 37°C. The plates were blocked with 5% nonfat dried milk in PBS for 1 hour and subsequently washed three times with PBS plus 0.2% Tween-20 and 5% nonfat dried milk. The plates were then be incubated with 200µl sera, diluted 1/100, from patients and normal controls, overnight at 4°C. The plates were washed and incubated with peroxidase-conjugated anti-human IgG (diluted 1/5000) for 1 hour. After washing, the presence of bound antibody was determined by incubating the wells with a 50mM citrate buffer solution containing o-phenylenediamine dihydrochloride (0.4mg/ml) (OPD, Sigma Chemical Co., St. Louis, MO) and measuring absorbance at 490nm.

### Results for Example 7

Using immunosorbent-purified antigens, microtiter plates for each assay were constructed. Sera from normal female controls (n=10), women with benign ovarian disease (n=10) and women with ovarian cancer (n=10 for each stage I-IV) at a 1:100 dilutions were incubated with the wells in duplicate overnight at 4°C. The well were washed with TBS three times and then incubated with peroxidase-conjugated anti-human IgG. The absorbance of each well was measured at 490nm. The mean absorbance of each patient was determined and plotted.

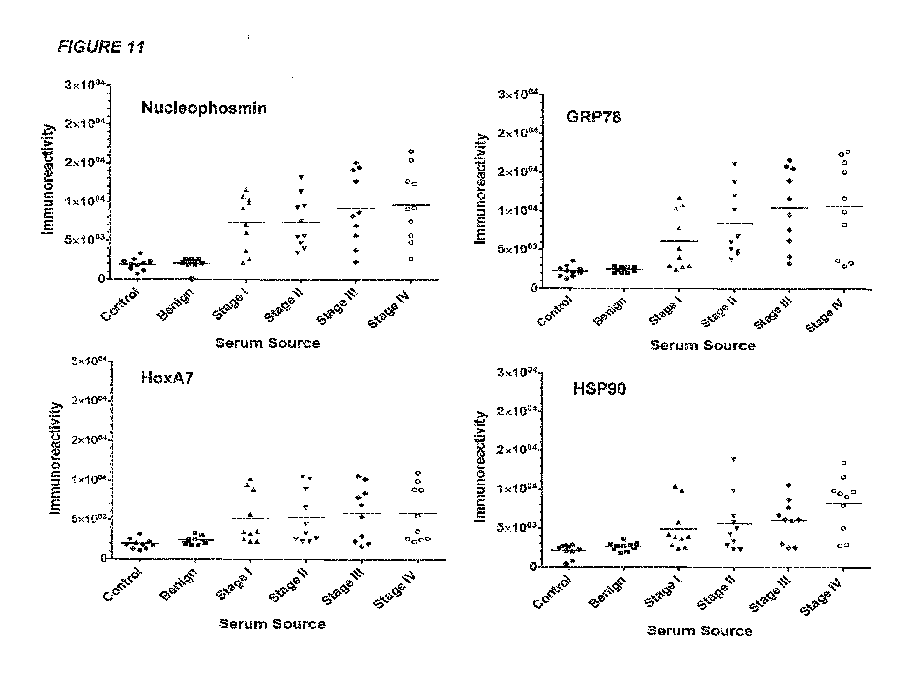

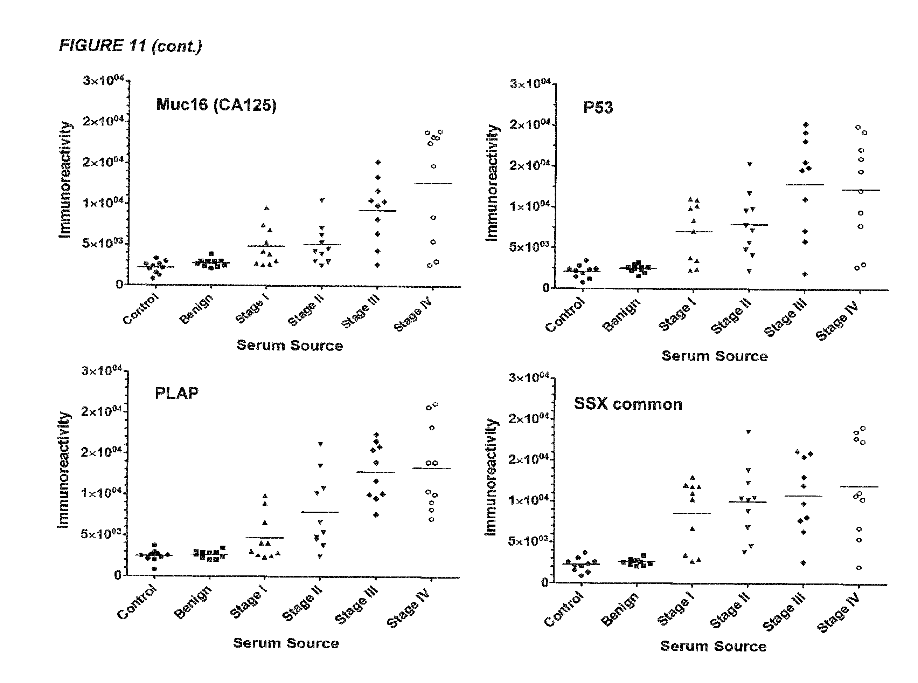

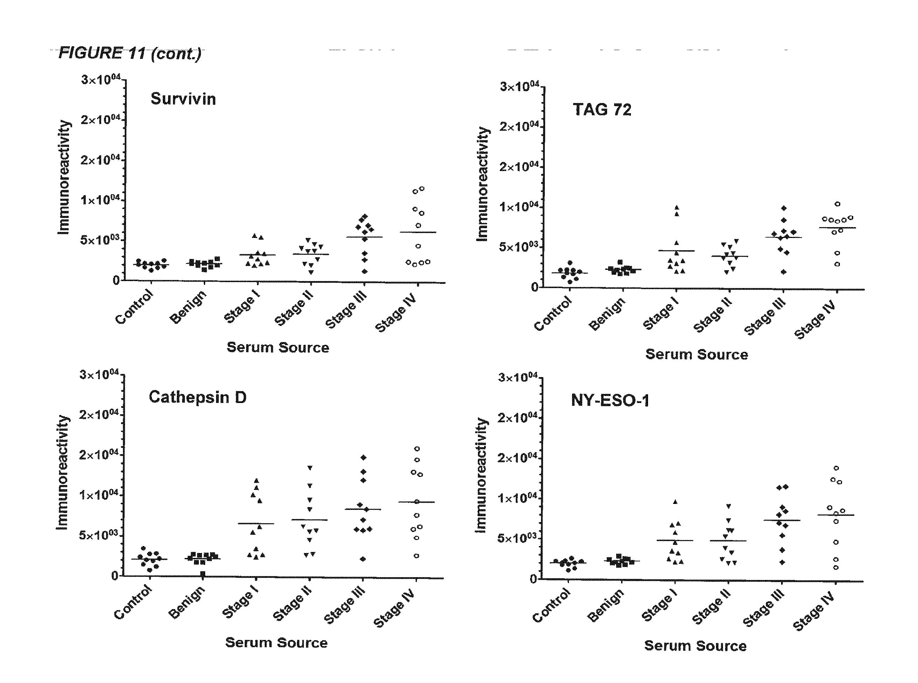

The immunoreactivity for both normal controls and women with benign disease were at the baseline and considered negative to all antigens tested (Figure 11). Each point on the graphs in Figure 11 represents the mean for each patient. In all cases, the means for the entire group were statistically different from the control and benign cases.

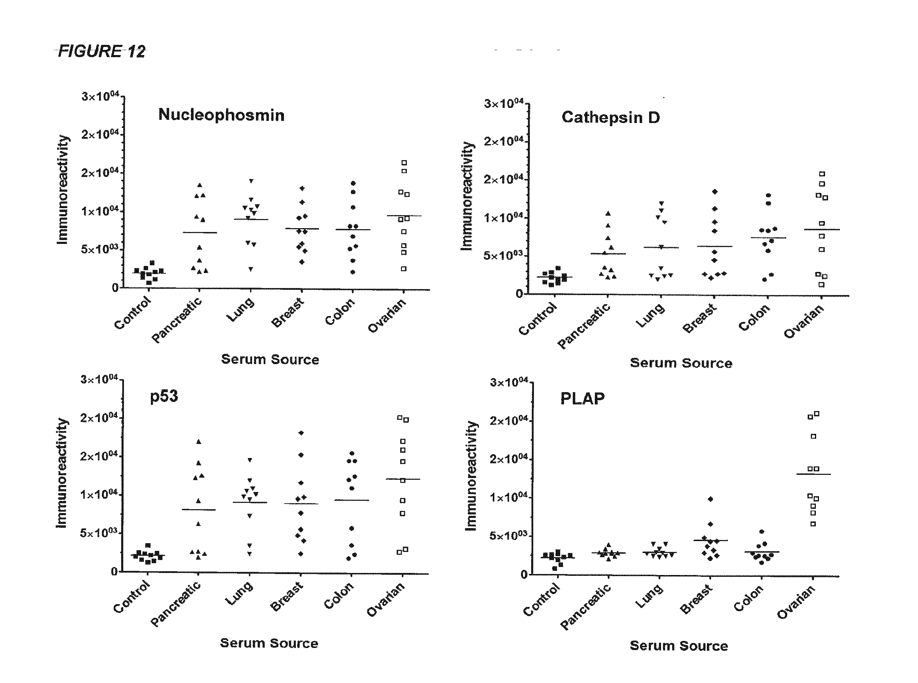

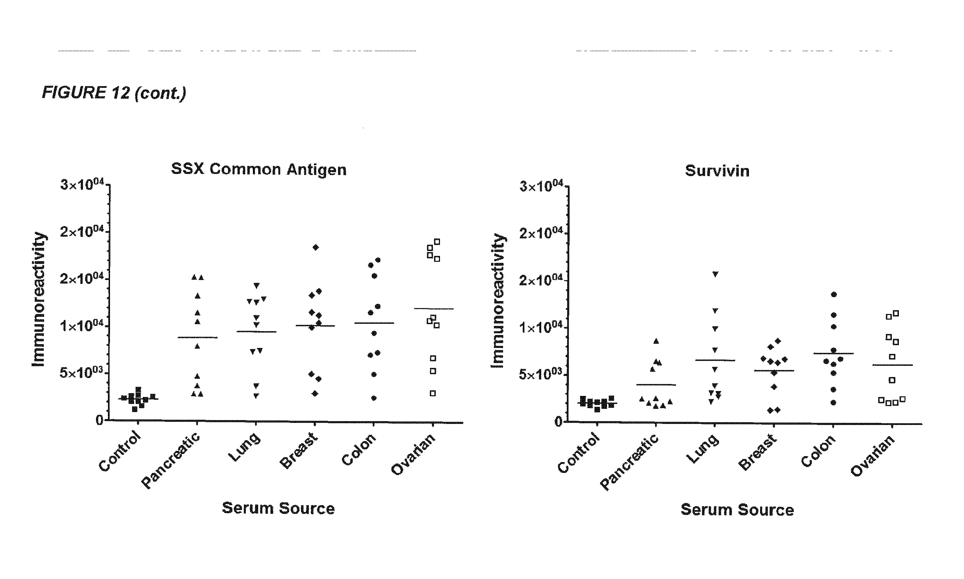

To assess the utility of the presently-disclosed methods as diagnostics for cancer in general, this study was repeated, except that sera from women with advanced pancreatic, lung, breast, and colon cancers were used. All cancer patients tested appeared to generate autoantibodies recognizing nucleophosmin, cathepsin D, p53, and SSX antigens. Only women with ovarian cancer recognized placental type alkaline phosphatase (PLAP). Patients with lung and colon cancer more strongly recognized survivin (Figure 12).

### EXAMPLE 8

### DEFINING ANTIGENIC EPITOPES ON RECOMBINANT VERSUS NATURAL PROTEINS

A portion (20ug) of each specific natural antigen and its recombinant counterpart were separated by sodium dodecyl sulfate-polyacrylamide gel electrophoresis (SDS-PAGE). After electrophoresis, the bands were visualized by Coomassie Blue staining and each band excised. The gels were equilibrated with SDS running buffer. The gel pieces containing the specific proteins were applied to the well of a 20% acrylamide gel. The gel pieces were chopped into smaller pieces and inserted in the sample well of the stacking gel for SDS-PAGE. One hundred ul of the electrode solution was added to the dried gel pieces. After incubation for 1 h, 20ul of diluted SDS sample buffer containing 10 ul of *Staphylococcus aureus* V8 protease (Pierce, Rockford, IL, USA) (0.1 ug/mL) in deionized water was overlaid on the sample solution. Electrophoresis was performed until the sample and protease were stacked in the upper gel, and interrupted for 1 h to digest the protein. Electrophoresis was then continued and the separated digests were electroblotted on PVDF membrane. The membranes were then incubated with patient serum, diluted 1:100, overnight. After washing 3X with TBS, the membranes were incubated for 1 hour with peroxidase-conjugated anti-human IgG as the secondary antibody. The bound immune complexes were visualized by enhanced chemiluminescence (ECL, Amersham Life Sciences, Arlington Heights, IL)

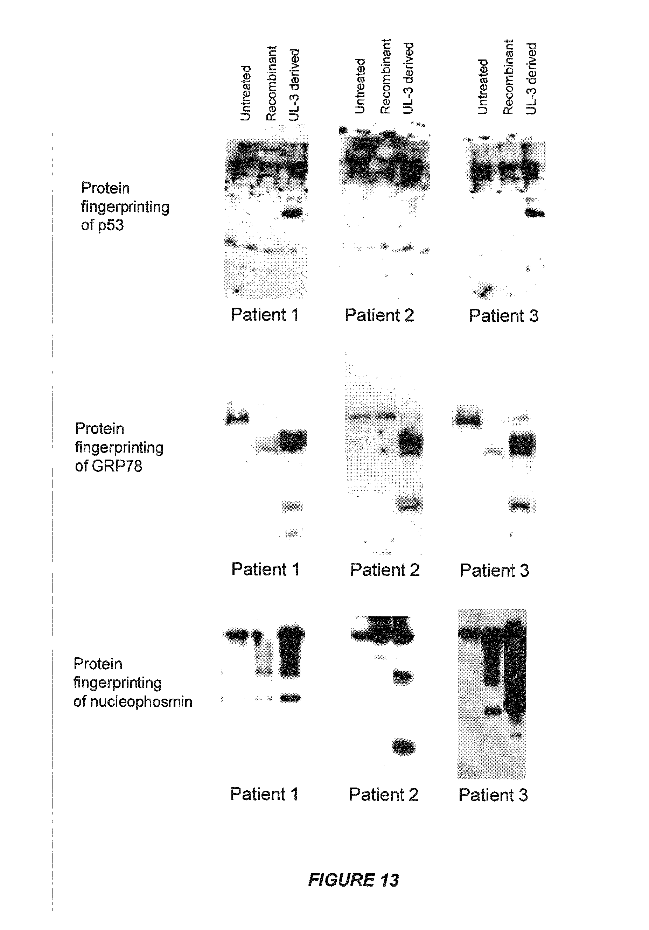

For p53, patients 1 and 3 both recognized an intermediate peptide band in the tumor-derived protein, which was not observed in the peptides of the recombinant protein (Figure 13). For GRP78, all patients recognized 3-5 additional peptide bands in the tumor-derived protein, which were not observed in the peptides of the recombinant protein (Figure 13). For nucleophosmin, all patients exhibited a more intense reactivity with the tumor-derived protein versus the recombinant protein (Figure 13). Patient 2 recognized an additional 3 bands in the tumor-derived nucleophosmin and patient 3 recognized 1 lower molecular weight band in comparison with the recombinant protein. These results demonstrate that the natural tumor-derived proteins derived from exosomes exhibit additional antigenic epitopes compared to recombinant proteins.

### EXAMPLE 9

### DIAGNOSTIC ASSAYS FOR INFERTILITY DISORDERS

To quantitate the level of immunoreactivity by infertility disorder patient-derived autoantibodies, the presence and reactivity of IgG within the sera of patients with endometriosis against cellular antigens derived from endometrial membrane, nucleus, and cytosol was quantitated by ELISA. Isolated proteins from each subcellular fraction were diluted 1:25 in a coupling buffer consisting of 100mM sodium carbonate and 0.5M NaCl, pH8.3. Aliquots were added to wells of a 96-well IMMULON 4^{™} microtiter plate (Chantilly, VA) and incubated overnight at 37°C. The plates were blocked with 5% nonfat dried milk in PBS for 1 hour and subsequently washed three times with PBS plus 0.2% Tween-20 and 5% nonfat dried milk. The plates were then be incubated with 200µl sera, diluted 1/100, from patients and normal controls, overnight at 4°C. The plates were washed and incubated with peroxidase-conjugated anti-human IgG (diluted 1/5000) for 1 hour. After washing, the presence of bound antibody was determined by incubating the wells with a 50mM citrate buffer solution containing o-phenylenediamine dihydrochloride (0.4mg/ml) (OPD, Sigma Chemical Co., St. Louis, MO) and measuring absorbance at 490nm.

Serum samples were obtained from women who were between 21-34 years of age and were diagnosed with infertility, resulting from endometriosis, premature ovarian failure (POF) and recurrent pregnancy loss. Control subjects were age-matched females with no pain symptoms, no pelvic anomalies and no previous pelvic surgery. Endometriosis was staged according to the revised American Society for Reproductive Medicine (rASRM) classification. The samples were obtained from the Divisions of Reproductive Endocrinology and Infertility of the Greenville Health System and of the University of Louisville. Twenty five specimens were obtained from controls, 25 patients each from women with Stage II endometriosis and Stage III endometriosis, 18 patients experiencing recurrent pregnancy loss, and 11 patients with premature ovarian failure. The samples were collected under an informed consented reviewed by the Human Studies Committee at GHS and the University of Louisville. Venous blood samples were obtained in heparinized tubes and the serum was isolated after clotting. The sera were stored at -70C until use.

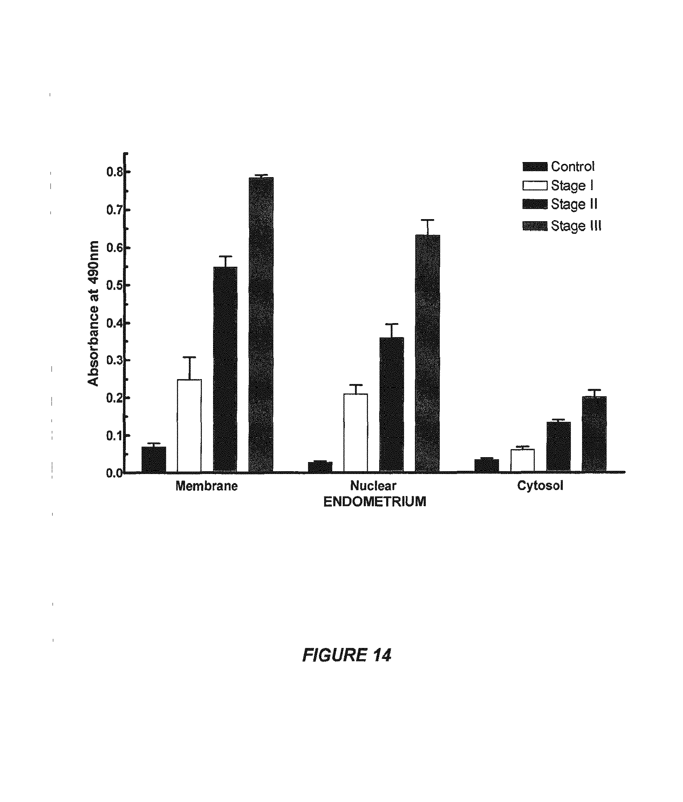

While variability was observed, all patients with infertility disorders tested exhibited a significant level of autoantibodies, in contrast to normal control patients (p<0.0001). Since patients exhibited an enhanced level of autoantibodies and the level of these autoantibodies exhibited significant variability, statistical comparisons were made between the relative absorbance for these IgG and stage of disease. The level of reactive IgG was observed to increase with stage of disease, with sera from stage II patients exhibiting a significant increase above normal sera (p<0.001) (Figure 14). The level of immunoreactivity present in stage III was significantly greater than that detected in the sera of stage II patients (p<0.01) for all antigen sources.

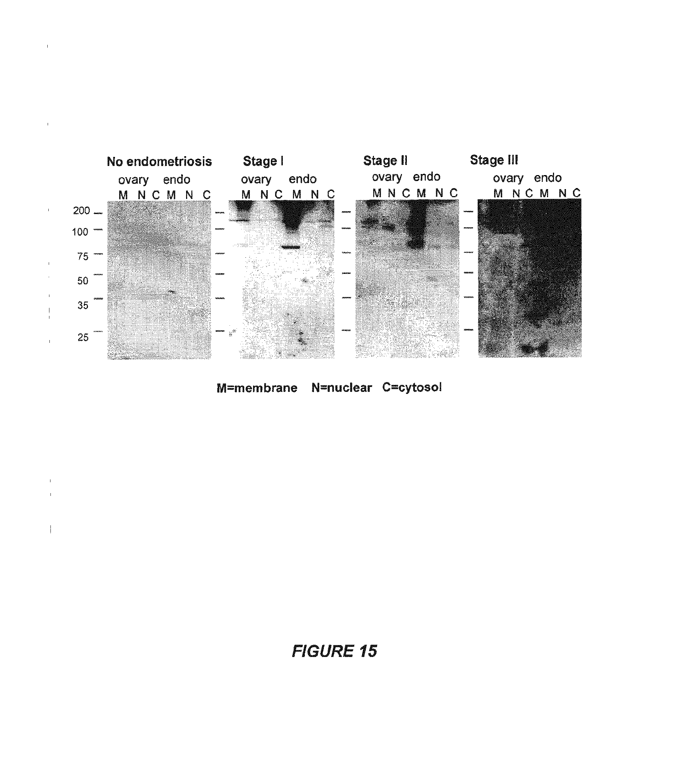

To characterize the reactivity of these autoantibodies with antigens from the endometrium, serum samples from normal controls and women with endometriosis (stages II and III) were further analyzed by Western immunoblotting. Representative immunoblots from the patient groups are presented in Figure 15. Control patients (without endometriosis) failed to exhibit significant recognition of any of the cellular antigens.

Patients with stage II endometriosis exhibited intense reactivity with cellular antigens from both the endometrium and ovary. Within these tissues, the strongest reaction was observed with antigens from the membrane fractions of the endometrium, followed by membrane antigens from the ovary. Sera from patients with Stage III endometriosis exhibited a similar pattern of reactivity; however, greater intense is observed was observed with membrane antigens from all sources. Within the endometrial membrane antigens, all patients with endometriosis recognized proteins at 80 and 140kD, while patients with stage III disease recognized additional bands at 10, 28, and 40kD. The common recognition of the 140kD membrane protein was shared with ovarian membrane antigens. In general, patients with endometriosis also exhibited strong shared recognition of 90 and 100kD proteins, while patients with stage III disease also recognized additional bands at 50 and 78kD. Little reactivity was observed with antigens derived from the cytosol.

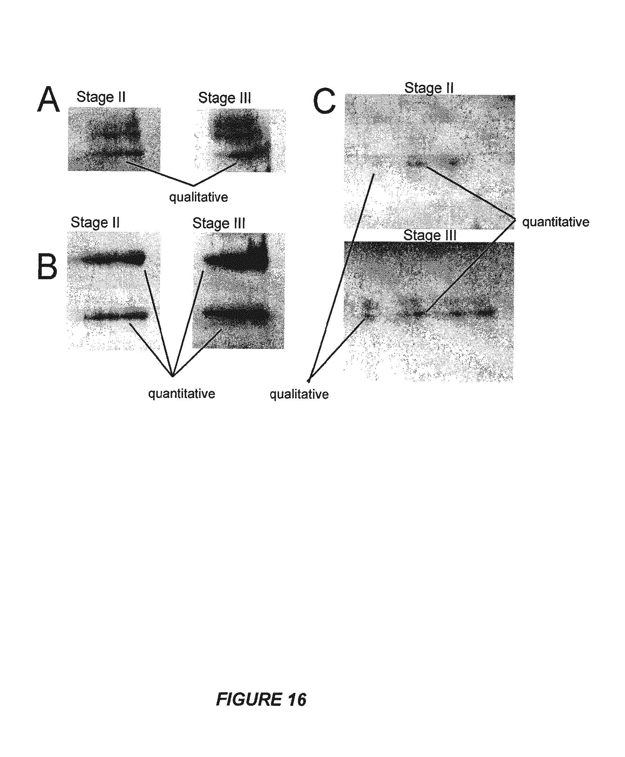

Due to the intensity of the reactivity, the antibody binding was further analyzed by separating the endometrial membrane antigens by two-dimensional electrophoresis (Figures 16A and 16B). This approach allowed the further separation of the membrane antigens. The increased level of reactivity observed in patients with stage III, versus stage II, endometriosis appears to result from both increased reactivity with the same components and the recognition of additional components.

Based on the findings of this study, assessment of endometrial autoantibodies to specific cellular proteins and/or suppression of NK activation can serve as diagnostic indicators of endometriosis. The presence of autoantibodies reactive with specific endometrial membrane antigens appears to be unique to endometriosis development and is correlated with stage of disease. The assessment of NK activation alone may not be specific for use of a diagnostic marker, but together with the presence of autoantibodies; they can provide the specificity and sensitivity for a definitive diagnosis of endometriosis.

To assess this approach to differentiate between etiologies of infertility, the protein array template consisted of 34 spots with 4 spots in width and 80 spots in length in a total size of 4x8cm. This template was used as a guide to spot solution onto MAGNAGRAPH membranes (MSI). To spot capture proteins onto the membranes, the template was placed on a light box and the MAGNAGRAPH membrane place on top of the template. Exosomes were isolated from the cultures of Hec-1 A endometrial cells. The isolated exosomes were solubilized and then precipitated using 0.25% SSA. The precipitated exosomal proteins were then resuspended in TBS and sonicated. This protein solution was then fractionated using a C18 reverse-phase HPLC column, eluting with a 0-100% acetonitrile gradient. The resulting 32 protein peaks were concentrated and the solvent removed by vacuum centrifugation. Each protein was resuspended in TBS and the protein concentration determined. Each protein solution (0.25µl) was manually loaded onto a single spot. Each protein solution was diluted to an initial concentration of 100µg/ml in bromophenol blue (for tracking). For each protein, spots of 2ng were made. The membranes were blocked with 5% BSA in 0.1M Tris-HCl, pH7.6, 0.15M NaCl (TBS) for 1 hour at room temperature. The membranes were then washed 3 times with TBS plus 0.1 % Tween-20 and 2 times with TBS. The membranes were then stored in sealed bags and refrigerated until use.

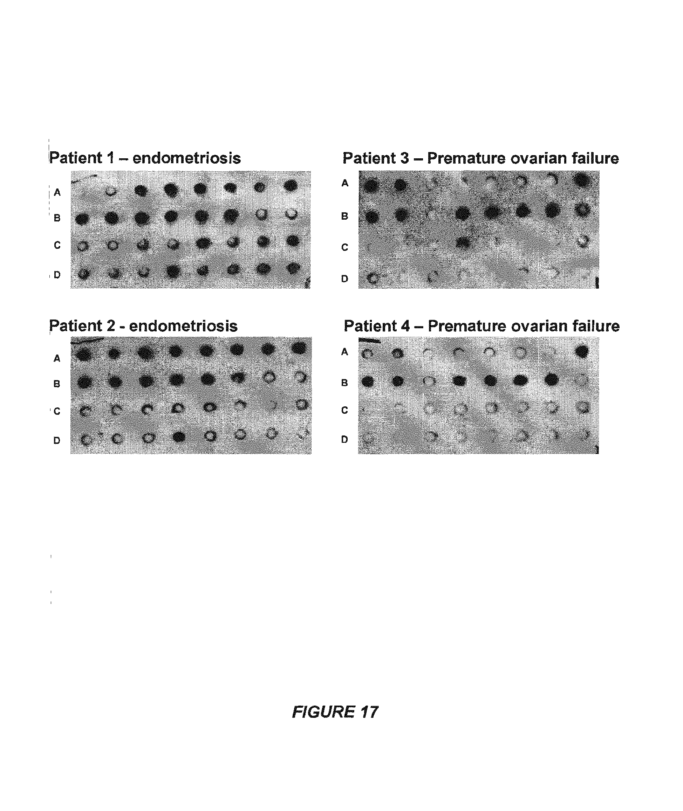

For diagnostic testing, the assay was performed with serum. Dilutions (1:100) of sera from known patients and normal fertile, non-pregnant controls were tested using the presently-disclosed protein arrays to define the optimal dilution of sera, combined with target protein dilutions, to identify patients with etiology-specific infertility. The array membranes were washed with TBS-Tween-20 prior to use and 10-fold serial dilutions of patient serum will be made in TBS. The diluted sera will be incubated with the membranes overnight at 4°C. The membranes were washed 3 times with TBS plus 0.1% Tween-20. The membrane was then be incubated with peroxidase-conjugated anti-human IgG, washed 3 times with TBS, and visualized by ECL. The resulting film was imaged with a Kodak D290 camera and analyzed using Kodak analysis software for spot recognition and quantitation (Figure 17). Distinct patterns of immunoreactivity were observed. Based on this differential recognition of endometrial protein antigens, patients can be distinguished and diagnosed with infertility disorders resulting from endometriosis versus those resulting premature ovarian failure.

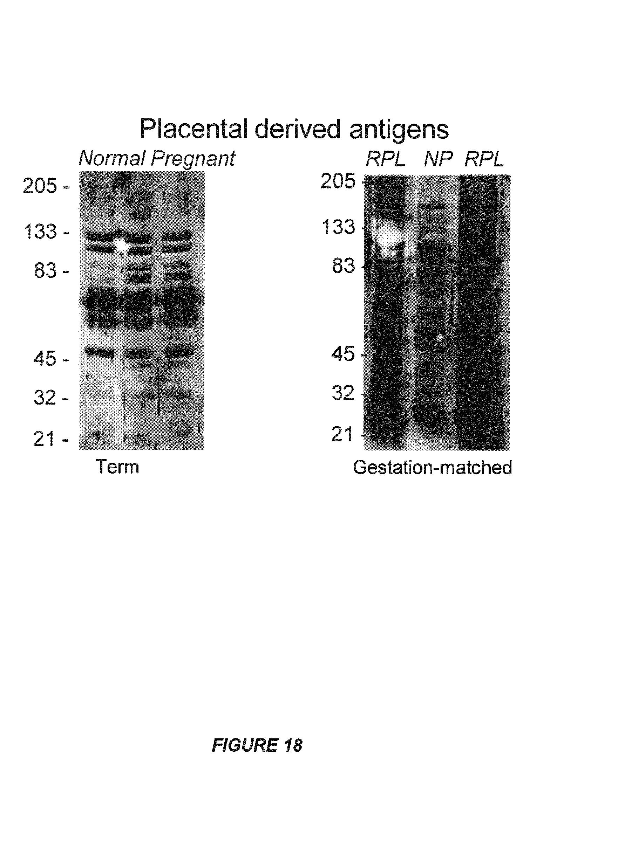

In a similar approach, women experiencing recurrent pregnancy losses were also examined for the presence of autoantibodies against placenta-derived antigens, using western immunoblotting (Figure 18). Pregnancy results in the production of autoantibodies, which result from the normal shift from a Th1 to Th2 immune response. However, women experiencing recurrent pregnancy loss exhibited both increased overall immunoreactivity and the recognition of unique components. Symptomatic patients, who subsequently experience a recurrent pregnancy loss, recognize additional antigens, at molecule weights less than 45,000 Daltons and greater than 130,000 Daltons.

### REFERENCES

Abu-Shakra et al., Cancer and autoimmunity: autoimmune and rheumatic features in patients with malignancies. Annals Rheum Dis 60:433-440, 2001.
Barbouche et al., Prognostic significance of autoantibodies to laminin in the sera of breast cancer patients. Europ J Clin Chem Clin Biochem 32: 511-514, 1994.
Brichory et al., Proteomics-based identification of protein gene product 9.5 as a tumor antigen that induces a humoral immune response in lung cancer. Cancer Res 61:7908-7912, 2001.
Canevari et al., Revised anti-cancer serological response: Biological significance and clinical implications. Annals Oncol 7:227-232, 1996.
Chinni et al., Cathepsin D and glucose-regulated protein 78 recognized by the humoral response of ovarian cancer patients. Clin Cancer Res 3:1557-1564, 1997.
Conroy et al., Autoantibodies to 90kD heat-shock protein in sera of breast cancer patients. Lancet 345:126, 1995.
Hellstrom et al., Principles of tumor immunity: Tumor antigens. In: DeVita VT, Hellman S, and Rosenberg SA, eds. Biologic therapy of cancer. New York: Lippincott, 1991: 35-52.
Jung and Schluesener, J Exp Med 173: 273-276, 1991.
Kotera Y et al., Humoral immunity against a tandem repeat epitope of human mucin muc-1 in sera from breast, pancreatic and colon cancer patients.
Kutteh et al., Immunologic characterization of tumor markers in human ovarian cancer cell lines. J Soc Gynecol Invest 3:216-222, 1996.
Labrecque et al., Analysis of the anti-p53 antibody response in cancer patients. Cancer Res 53:3468-3471, 1993.
Liotta et al., Proteomic patterns in sera serve as biomarkers of ovarian cancer. Gynecol Oncol 88: S25-S28, 2003.
Lubin et al., Analysis of p53 antibodies in patients with various cancers define B-cell epitopes of human p53: Distribution on primary structure and exposure on protein surface. Cancer Res 53:5872-5876, 1993.
Merimsky et al., Antigens and antibodies in malignant melanoma. Tumour Biol 15:188-202, 1994.
Nakamura et al. Handbook of Experimental Immunology (4th Ed.), Weir et al. (eds). Vol. 1, Chapter 27, Blackwell Scientific Publ., Oxford, 1987.
Niklinska et al., Strong association between p53 protein accumulation, serum antibodies, and gene mutation in non-small cell lung cancer. Folia Histochem Cytobiol 39:51-56, 2001.
Old LJ, Chen YT: New paths in human cancer serology. J Exp Med 187:1163-1167, 1998.
Peoples et al., Breast and ovarian cancer specific cytotoxic T lymphocytes recognize the same HER2/neu-derived peptide. Proc Natl Amer Sci USA 92: 432-436, 1995.
Taylor and Doellgast, Quantitation of peroxidase-antibody binding to membrane fragments using column chromatography. Analytical Biochemistry, 98:53-59, 1979.
Taylor et al., Identification of antigenic components recognized by membrane bound" antibodies from ovarian cancer patients. Am J Reprod Immunol 6:179-184, 1984.
U.S. Patent No. 3,817,837.
U.S. Patent No. 3,850,752.
U.S. Patent No. 3,939,350.
U.S. Patent No. 3,996,345.
U.S. Patent No. 4,275,149.
U.S. Patent No. 4,277,437
U.S. Patent No. 4,302,534.
U.S. Patent No. 4,366,241.
U.S. Patent No. 4,637,988.
U.S. Patent No. 4,786,594.
U.S. Patent No. 5,108,896.
U.S. Patent No. 5,229,302.
U.S. Patent No. 5,629,164.
U.S. Patent No. 5,691,154.
Vlock et al, Incidence of serum antibody reactivity to autologous head and neck cancer cell lines and augmentation of antibody reactivity following acid dissociation and ultrafiltration. Cancer Res 49:1361-1365, 1989.
Vogl et al., Autoimmunity against p53 predicts invasive cancer with poor survival in patients with ovarian mass. Brit J Cancer 83:1338-1343, 2000.
Winter et al., Development of antibodies against p53 in lung cancer patients appears to be dependent on the type of p53 mutation. Cancer Res 52: 4168-4174, 1992
Yamamoto et al., Infrequent presence of anti-c-myc antibodies and absence of c-myc oncoprotein in sera from lung cancer patients. Oncology 56:129-133, 1999.

It will be understood that various details of the presently disclosed subject matter can be changed without departing from the scope of the presently disclosed subject matter. Furthermore, the foregoing description is for the purpose of illustration only, and not for the purpose of limitation.

### Clauses

1. A method for diagnosing a disorder associated with autoantibody production in a subject, comprising:
   (a) providing a biological sample comprising or suspected of comprising autoantibodies from a subject;
   (b) contacting an antigen with the sample, wherein the antigen comprises an autoantibody immunoreactive peptide isolated from an exosome;
   (c) detecting autoantibodies in the sample immunoreactive to the antigen; and
   (d) comparing a level of autoantibody immunoreactivity to the antigen with a reference level to diagnose the disorder in the subject.
2. The method of clause 1, wherein the disorder is a cancer or an infertility disorder.
3. The method of clause 2 wherein the disorder is an epithelial cancer or an adenocarcinoma.
4. The method of clause 3, wherein the antigen comprises a cancer antigen peptide selected from the group consisting of a tumor suppressor family peptide, a nucleic acid binding peptide, an anti-apoptotic peptide, an oncogene family peptide, a homeobox peptide, a cancer testis antigen peptide, a heat shock protein family peptide, an enzyme precursor of pro-lysosomal enzyme family peptide, PLAP, an adhesion molecule family peptide, an adhesion related peptide, and a kinase family peptide.
5. The method of clause 2, wherein the disorder is an infertility disorder selected from the group consisting of premature ovarian failure (POF), polycystic ovary syndrome (PCOS), endometriosis, preeclampsia, preterm birth, intrauterine growth restriction, and recurrent pregnancy loss.
6. The method of clause 1, wherein the biological sample comprises milk, blood, serum, plasma, ascites, cyst fluid, tears, urine, saliva, tissue, or combinations thereof.
7. The method of clause 1, wherein the subject is a mammal.
8. The method of clause 1, wherein the exosome is isolated from a cell.
9. The method of clause 8, wherein the cell is a cultured cell.
10. The method of clause 9, wherein the cell is a cancer cell.
11. The method of clause 10, wherein the cancer cell is an ovarian cancer cell, a cervical cancer cell, a breast cancer cell, an endometrial cancer cell, a colon cancer cell, a prostate cancer cell, a lung cancer cell, a melanoma cell, a pancreatic cancer cell, or a choriocarcinoma cell.
12. The method of clause 10, wherein the cell is a UL-1 cell, a UL-2 cell, a UL-3 cell, or a UL-6 cell.
13. The method of clause 9, wherein the cell a placental cell.
14. The method of clause 1, wherein the detecting comprises a technique selected from the group consisting of ELISA, RIA, multiplex immunoassay, immunoprecipitation and Western blotting.
15. A method for characterizing a disorder associated with autoantibody production in a subject, comprising:
   (a) providing a biological sample comprising or suspected of comprising autoantibodies from a subject;
   (b) contacting an antigen with the sample, wherein the antigen comprises an autoantibody immunoreactive peptide isolated from an exosome;
   (c) detecting autoantibodies in the sample immunoreactive to the antigen; and
   (d) quantitating a level of autoantibody immunoreactivity to the antigen to thereby characterize the disorder in the subject.
16. The method of clause 15, wherein the disorder is a cancer or an infertility disorder.
17. The method of clause 16 wherein the disorder is an epithelial cancer or an adenocarcinoma.
18. The method of clause 17, wherein the antigen comprises a cancer antigen peptide selected from the group consisting of a tumor suppressor family peptide, a nucleic acid binding peptide, an anti-apoptotic peptide, an oncogene family peptide, a homeobox peptide, a
   cancer testis antigen peptide, a heat shock protein family peptide, an enzyme precursor of pro-lysosomal enzyme family peptide, PLAP, an adhesion molecule family peptide, an adhesion related peptide, and a kinase family peptide.
19. The method of clause 16, wherein the disorder is an infertility disorder selected from the group consisting of premature ovarian failure (POF), polycystic ovary syndrome (PCOS), endometriosis, preeclampsia, preterm birth, intrauterine growth restriction, and recurrent pregnancy loss.
20. The method of clause 15, wherein the biological sample comprises milk, blood, serum, plasma, ascites, cyst fluid, tears, urine, saliva, tissue, or combinations thereof.
21. The method of clause 15, wherein the subject is a mammal.
22. The method of clause 15, wherein the exosome is isolated from a cell.
23. The method of clause 22, wherein the cell is a cultured cell.
24. The method of clause 23, wherein the cell is a cancer cell.
25. The method of clause 24, wherein the cancer cell is an ovarian cancer cell, a cervical cancer cell, a breast cancer cell, an endometrial cancer cell, a colon cancer cell, a prostate cancer cell, a lung cancer cell, a melanoma cell, a pancreatic cancer cell, or a choriocarcinoma cell.
26. The method of clause 24, wherein the cell is a UL-1 cell, a UL-2 cell, a UL-3 cell, or a UL-6 cell.
27. The method of clause 23, wherein the cell a placental cell.
28. The method of clause 15, wherein the detecting comprises a technique selected from the group consisting of ELISA, RIA, multiplex immunoassay, immunoprecipitation and immunoblotting.
29. The method of clause 16, wherein the disorder is a cancer and characterizing the disorder comprises determining a stage of the cancer.
30. A method for detecting and/or quantitating a level of autoantibodies in a subject, comprising:
   (a) providing a biological sample comprising or suspected of comprising autoantibodies from a subject;
   (b) contacting an antigen with the sample, wherein the antigen comprises an autoantibody immunoreactive peptide isolated from an exosome; and
   (c) detecting and/or quantitating a level of the autoantibodies in the sample immunoreactive to the antigen.
31. The method of clause 30, wherein the autoantibodies are associated with a cancer or an infertility disorder.
32. The method of clause 31, wherein the cancer is an epithelial cancer or an adenocarcinoma.
33. The method of clause 32, wherein the antigen comprises a cancer antigen peptide selected from the group consisting of a tumor suppressor family peptide, a nucleic acid binding peptide, an anti-apoptotic peptide, an oncogene family peptide, a homeobox peptide, a cancer testis antigen peptide, a heat shock protein family peptide, an enzyme precursor of pro-lysosomal enzyme family peptide, PLAP, an adhesion molecule family peptide, an adhesion related peptide, and a kinase family peptide.
34. The method of clause 31, wherein the disorder is an infertility disorder selected from the group consisting of premature ovarian failure (POF), polycystic ovary syndrome (PCOS), endometriosis, preeclampsia, preterm birth, intrauterine growth restriction, and recurrent pregnancy loss.
35. The method of clause 30, wherein the biological sample comprises milk, blood, serum, plasma, ascites, cyst fluid, tears, urine, saliva, tissue, or combinations thereof.
36. The method of clause 30, wherein the subject is a mammal.
37. The method of clause 30, wherein the exosome is isolated from a cell.
38. The method of clause 37, wherein the cell is a cultured cell.
39. The method of clause 38, wherein the cell is a cancer cell.
40. The method of clause 39, wherein the cancer cell is an ovarian cancer cell, a cervical cancer cell, a breast cancer cell, an endometrial cancer cell, a colon cancer cell, a prostate cancer cell, a lung cancer cell, a melanoma cell, a pancreatic cancer cell, or a choriocarcinoma cell.
41. The method of clause 39, wherein the cell is a UL-1 cell, a UL-2 cell, a UL-3 cell, or a UL-6 cell.
42. The method of clause 38, wherein the cell a placental cell.
43. The method of clause 30, wherein the detecting comprises a technique selected from the group consisting of ELISA, RIA, multiplex immunoassay, immunoprecipitation and Western blotting.
44. A kit for detecting autoantibodies in a sample, comprising an autoantibody immunoreactive peptide antigen and a container for containing the antigen, wherein the antigen is isolated from an exosome.
45. The kit of clause 44, wherein the antigen is attached to a support.
46. The kit of clause 45, wherein the support is a microtiter plate, a membrane, a polystyrene bead, a test tube or a dipstick.
47. The kit of clause 44, comprising an antibody preparation that binds to an autoantibody.
48. The kit of clause 47, wherein the antibody preparation comprises a detectable label.
49. The kit of claim 48, wherein the detectable label comprises a radiolabel, an enzyme, biotin, a dye, a fluorescent tag label, a hapten or a luminescent label.
50. A method for diagnosing a fertility disorder in a subject, comprising:
   (a) providing a biological sample comprising or suspected comprising autoantibodies associated with a fertility disorder from a subject;
   (b) contacting at least one antigen with the sample, wherein the antigen comprises a peptide antigen that binds autoantibodies associated with the fertility disorder and is selected from the group consisting of nuclear antigens with molecular weights of about 50kD and 80kD and membrane antigens with molecular weights of about 10kD, 30kD, 45kD, 90kD and 125kD;
   (c) detecting autoantibodies in the sample immunoreactive to the antigen; and
   (d) comparing a level autoantibody immunoreactivity to the antigen with a reference level to diagnose the fertility disorder and/or predict a risk for developing the fertility disorder in the subject.
51. The method of clause 50, wherein the infertility disorder is selected from the group consisting of premature ovarian failure (POF), polycystic ovary syndrome (PCOS), endometriosis, preeclampsia, preterm birth, intrauterine growth restriction, and recurrent pregnancy loss.
52. The method of clause 50, wherein the biological sample comprises milk, blood, serum, plasma, ascites, cyst fluid, pleural fluid, tears, urine, saliva, tissue, or combinations thereof.
53. The method of clause 50, wherein the autoantibodies are reactive to ovary, endometrium, placenta, or combinations thereof.
54. The method of clause 50, wherein the subject is a mammal.
55. The method of clause 50, wherein the antigen is isolated from a cultured cell.
56. The method of clause 55, wherein the cultured cell is a placental cell.
57. The method of clause 50, wherein the antigen is isolated from placenta, ovary, endometrium, or combinations thereof.
58. The method of clause 50, wherein the detecting comprises a technique selected from the group consisting of ELISA, RIA, multiplex immunoassay, immunoprecipitation and Western blotting.

## Claims

1. A method for characterizing a disorder in a subject, comprising:
contacting a biological sample from the subject with an immunoreactive agent capable of binding a polypeptide present on the surface of an extracellular vesicle, wherein the polypeptide is capable of eliciting an autoimmune response in the subject;
detecting a reaction between the immunoreactive agent and the polypeptide; and
comparing the reaction to a reference level to characterize the disorder in the subject.

2. The method of claim 1, wherein the disorder is cancer.

3. The method of claim 2, wherein the cancer is epithelial cancer or adenocarcinoma.

4. The method of claim 1, wherein the polypeptide comprises a tumor-associated antigen.

5. The method of claim 1, wherein the polypeptide comprises a tumor-specific mutation or post-translational modification.

6. The method of claim 1, wherein the polypeptide is MUC-1, MUC-16, TAG-72, NY-ESO-1, SSX-1, SSX-2, SSX-4, SCP-1, HSP27, HSP60, HSP90, GRP78, mesothelin, p53, p63, p73, MOC31, cytokeratin, nucleolin, nucleophosmin, c-myc, c-ras, c-erb2, mdm2, Hox-A7, Hox-B7, procathepsin D, PLAP, EpCAM, CA125, EGF kinase or a combination thereof.

7. A kit comprising one or more immunoreactive agent capable of binding a polypeptide present on the surface of an extracellular vesicle, wherein the polypeptide is capable of eliciting an autoimmune response in a subject.

8. The kit of claim 7, wherein the polypeptide comprises a tumor-associated antigen.

9. The kit of claim 7, wherein the polypeptide comprises a tumor-specific mutation or post-translational modification.

10. The kit of claim 7, wherein the polypeptide is MUC-1, MUC-16, TAG-72, NY-ESO-1, SSX-1, SSX-2, SSX-4, SCP-1, HSP27, HSP60, HSP90, GRP78, mesothelin, p53, p63, p73, MOC31, cytokeratin, nucleolin, nucleophosmin, c-myc, c-ras, c-erb2, mdm2, Hox-A7, Hox-B7, procathepsin D, PLAP, EpCAM, CA125, EGF kinase or a combination thereof.
